Europäisches Patentamt

European Patent Office

Office européen des brevets

(19)

(11) Publication number: **0 228 457 B1**

(12) # EUROPEAN PATENT SPECIFICATION

(45) Date of publication of patent specification: **26.08.92**  (51) Int. Cl.[5]: **C12N 1/20**

(21) Application number: **86904587.2**

(22) Date of filing: **30.06.86**

(86) International application number:
**PCT/US86/01375**

(87) International publication number:
**WO 87/00194 (15.01.87 87/01)**

(54) **EMERGENCE-PROMOTING RHIZOBACTERIA.**

(30) Priority: **02.07.85 CA 486217**

(43) Date of publication of application:
**15.07.87 Bulletin 87/29**

(45) Publication of the grant of the patent:
**26.08.92 Bulletin 92/35**

(84) Designated Contracting States:
**BE DE FR GB IT SE**

(56) References cited:
**EP-A- 0 040 110**
**EP-A- 0 064 720**
**EP-A- 0 157 351**
**EP-A- 0 227 336**
**FR-A- 2 193 085**

(73) Proprietor: **IMPERIAL OIL LIMITED**
**111 St. Clair Avenue West**
**Toronto Ontario, M5W 1K3(CA)**

(72) Inventor: **KLOEPPER, Joseph, Wayne**
**12 Mullen Place**
**Georgetown, Ontario L7G 2R9(CA)**
Inventor: **SCHER, Frances, Murphy**
**43, Finchley Crescent**
**Bramalea, Ontario L6T 3P5(CA)**

(74) Representative: **Eyles, Christopher Thomas et al**
**W.P. THOMPSON & CO. High Holborn House**
**52-54 High Holborn**
**London WC1V 6RY(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

BIOLOGICAL ABSTRACTS, vol. 81, 1986, abstract no. 29561, Biological Abstracts Inc., Philadelphia, PA, US; J.W.F. KLOEPPER et al.: "Measuring the spermosphere colonizing capacity (spermosphere competence) of bacterial inoculants", & CAN. J. MICROBIOL. 31(10): 926-929, 1985

BIOLOGICAL ABSTRACTS, vol. 81, no. 4, 1986, abstract no. 77652, Biological Abstracts Inc., Philadelphia, PA, US; L. RAN et al.: "Nitrogen-fixing pseudomonads isolated from roots of plants grown in the Canadian High Arctic", & APPL. ENVIRON. MICROBIOL. 51(2), 251-255, 1986

CAN. J. MICROBIOL., vol. 30, 1984, pages 151-157; F.M. SCHER et al.: "A method for assessing the root-colonizing capacity of bacteria on maize"

ANNUAL REVIEW OF PHYTOPATHOLOGY, vol. 12, 1974, pages 181-197; M.E. BROWN: "Seed and root bacterization"

BIOLOGICAL ABSTRACTS, vol. 85, no. 7, abstract no. 64316, Biological Abstracts Inc., Philadelphia, PA, US; J.W. KLOEPPER et al.: "Plant growth-promoting rhizobacteria on canola (rapeseed), & PLANT DIS. 72(1): 42-46, 1988

Mikrobiologiya, vol. 21, n 10, issued 1981 (Moscow, USSR) O. Berestetsky et al "Utilization by soil microorganisms of volatile organic compounds liberated by germinating seeds as a source of carbon and energy", pages 898-902

Canadian Journal of Microbiology, vol. 31, issued June 1985 (Ottawa, Canada), F. Scher et al "Chemotaxis of fluorescent Pseudomonas spp. to soybean seed exudates in vitro and in soil" pages 570-574

Phytopathology, vol. 71, issued 1981, J. Kloepper et al "Plant growth-promoting Rhizobacteria and plant growth under gnotobiotic conditions" pages 642-644

Soil Biology and biochemistry, vol. 17, n 6, issued October 1985 (Oxford, England) J. Kipe-Nolt et al "Root exudation of sorghum and utilization of exudates by nitrogen-fixing bacteria", pages 859-63

Canadian Journal of Microbiology, vol. 21, issued 1975 (Ottawa, Canada) S. Schenck et al "Effect on microorganisms of volatile compounds release from germinating seeds", pages 1622-34

**Description**

This invention relates to psychrotrophic bacteria, and their use in promotion of growth of economically significant plants. More particularly, it pertains to a certain group of newly discovered and newly isolated psychrotrophic bacteria and their use in promoting the growth of, for example, soybean (Glycine max) and canola (rapeseed) (Brassica campestris and B. napus) plants.

BACKGROUND OF THE INVENTION

Evidence suggests that certain bacteria are able to enhance plant growth, at least under laboratory or simulated field conditions. These plant growth promoting bacteria typically are those which occupy the rhizosphere of the growing plant and exert some favourable influence in that region. Accordingly, these bacteria may be utilized as inoculants applied in a manner which permits their occupation of the growth environment of the plant root such as by seed or soil inoculation prior to planting or during or after planting.

Specific strains of these root-colonizing bacteria, termed plant growth-promoting rhizobacteria (PGPR), have recently been used as experimental inoculants to increase yield or such root or tuber crop plants as sugar beet (Suslow and Schroth, 1982), radish (Kloepper and Schroth, 1978) and potato 'Burr, et. al., 1978; Howie and Echandi, 1983; Kloepper, et. al., 1980). In each case, evidence of enhanced plant growth was noted during the growing season after emergence but before crop maturity. With potato, increased early-season plant development was manifested by increased stolon lengths on PGPR-treated plants (Kloepper et. al. 1980; Kloepper and Schroth, 1981A; Kloepper and Schroth, 1981B). With sugar beet, the earliest indication of plant growth promotion with PGPR was a significant increase in plant weight after seedling establishment (Suslow and Schroth, 1982); neither seedling emergence nor early seedling vigour were affected by PGPR treatments.

It is believed that some plant growth-promoting rhizobacteria respond in a positive chemotactic way to root exudates and, once attracted, colonize the plant rhizosphere explosively. In this way, they occupy the rhizosphere and the nutrients available thereat, inhibiting subsequent colonization by plant pathogens. It appears that once commercial application of these rhizobacteria is realized, the result will be enhanced crop yield.

There remains, however, a need for a method and means by which emergence of a crop can be promoted particularly for use in those areas where climate retards the growing season, and consequently reduces the opportunity for stands to reach maturity in the time available for growth. An objective of this invention is to provide such a method such that the crop or seed treatment selected according to the procedures contained herein has the unexpected and valuable benefit.

SUMMARY OF THE INVENTION

In its broadest aspect the present invention relates to bacteria which are capable of promoting plant emergence i.e. of promoting emergence of seedlings from seed, particularly seed of agricultural importance. More particularly, it has now been observed that some bacterial strains will reproducibly induce increases in seedling emergence of up to 100% greater than controls, in the field. Moreover, these bacteria are capable of growth under conditions favourable to psychrotophic bacteria, a characteristic of particular importance given the cold environments in which they can be applied. For example, the bacteria of the present invention will be useful at the extreme northern perimeter of a crop zone. More particularly, canola on the Canadian prairies is concentrated in areas with 90 or more frost free days. It bacteria-induced acceleration in seedling emergence translates into crop maturity or even five days sooner, the total hectarage available for canola growth would be increased substantially.

The bacteria of the present invention, herein designated emergence promoting rhizobacteria or "EPR" for brevity, are represented by a seemingly diverse collection of genera, including Pseudomonas, Serratia,and Beijerincka. All may be isolated according to the screening process described generally and in detail herein and all, therefore, share certain common characteristics. The EPR of the present invention are psychrotrophic rhizobacteria and may therefore be recovered from plant roots or surrounding soil from artifically or naturally cold climates. A further unique feature of the bacteria is their ability to utilize plant seed exudate as the sole carbon and nitrogen source.

As used herein, "psychrotrophic" is used to describe bacteria which have the ability to multiply at 0-5°C, as the term is defined in "An ecological study of the psychrotrophic bacteria of soil, water, grass and hay", R.G. Druce and S.B. Thomas, 1970, J. Appl. Bact. 33:420-425. It is to be noted however, that unlike psychrophilic bacteria, the psychrotrophs are also able to grow at higher temperatures e.g. ambient.

The screening process by which these EPR are identified and isolated is a more determinative measure of defining those bacteria falling within the scope of the invention, however. According to this aspect of the invention, rhizobacteria exhibiting psychrotrophic growth and growth on seed-exudate containing medium are selected for further screening in a bioemergence assay described herein. Those bacteria which meet or surpass bioemergence assay criteria are identified as EPR. Thus, there is provided a method for identifying bacteria capable of promoting emergence of seedlings from plant seed when applied to the growth environment of the seed, which comprises collecting bacteria which colonize plant roots;

selecting those collected bacteria which exhibit growth under psychrotropic conditions and growth on media containing seed exudate as the sole carbon source;

growing seedlings from a population of seed grown in soil at temperatures tolerable by psychrotrophic bacteria wherein the growth environment of an experimental portion of the seed population contains an effective population of one of the selected bacteria and the growth environment of the remaining portion contains no selected bacteria; and

selecting those bacteria added to the growth environment of the experimental seed population exhibiting an at least 50% greater number of emerged seedlings by comparison with the control population each day for at least three consecutive days during the seedling emergence period.

The bacteria of the present invention are defined as those bacteria which meet the criteria and endure the screening and selection process defined above. It will be appreciated by those skilled in the art that bacteria additional to those EPR strains identified specifically herein will be useful as emergence promoters. Accordingly, the scope of the invention should not be construed as limited thereto.

The present invention may be practised in a variety of ways. For example, crop seeds may be treated with a composition containing one or more EPR according to the invention, prior to planting. Thus the seeds may be inoculated with a liquid composition or the EPR, or surface treated with such a liquid, solid or semi-solid composition, e.g. by dipping, soaking, spraying, dusting, etc. or by applying a peat based mixture as is common in Rhizobium inoculants industry. Alternatively, the soil environment in which the plants are grown may be treated with liquid, solid or semisolid compositions containing the EPR in effective amounts, either before, in conjunction with or after planting. Such application may be by liquid or solid application. In situations where transplantation takes place, the root structure or other part of the plant may be inoculated or surface treated with such liquid, semi-solid or solid compositions during transplantation, or the new rooting medium may be appropriately treated with the EPR composition. Best results have been obtained herein using inocula which deliver a bacterial population or $10^3$ to $10^7$ cells to the spermosphere of an individual seed although a broader range of $10^2$ to $10^9$ has been experimentally useful. Seed bearing such a population and compositions, containing EPR and an agronomically acceptable carrier, which deliver such populations to the spermosphere represent another significant aspect of the invention.

A further, significant aspect of the present invention resides in a process for promoting the growth of agricultural crops which comprises applying to the growth environment of the crop an effective amount of at least one viable psychrotrophic EPR bacterium said bacterium belonging preferably to the genera Pseudomonas, Serratia or Beijerinckia.

While the EPR are particularly useful in cold climates owing to their ability to grow at cold temperatures, it will be appreciated that they are equally useful in warmer climes. Accordingly, the process aspect is not limited specifically to applications in certain climatic zones.

Further, it will be apparent herein that appropriate use of these bacteria results not only in promotion of seedling emergence but can ultimately result in enhanced crop yield.

The emergence-promoting rhizobacteria according to the present invention may be utilized by methods known in the art such, for example, as direct coating on seeds say by freeze-drying or in formulations including agronomically acceptable adjuvants and carrier normally employed for facilitating the dispersion of active ingredients for agricultural applications.

The precise formulation, dosage, made of application and other variables are chosen to enhance the novel rhizobacteria in any given application. Thus, the previously described novel microorganisms may be formulated as a suspension or a dispersion, an aqueous or non-aqueous medium, as a dust, as a wetable powder, as an emolsifiable concentrate, as a granule, or as any of several known types of formulations depending on the mode of application. These combinations may be applied as sprays, dusts or granules to the seeds, seed pieces, roots, plants, soil or planting site at which such activity is desired.

## DETAILED DESCRIPTION OF THE PREFERRED EMBODIMENTS

The EPR of the present invention are preferably obtained from one of two sources; plants growing naturally in cold climates may be used as the EPR source or plants grown from surface-sterilized seed in soil obtained from cola climates may be used. In either case, it is preferred to isolate the bacteria which have colonized the rhizosphere by plating at ambient or cooler temperatures both roots and bacteria on a medium which takes advantage of bacterial chemotaxis to draw the bacteria from the root. Asparagine soft agar (ASA) is an example of one such medium. This medium has been adapted from seed exudate soft agar (Scher et al, 1985) and may be a partial indicator of root colonization capacity. Bacteria which grow out from the root segments may then be plated on standard bacterial agar although Pseudomonas agar F (PAF) is preferred in this particular art.

In screening the collected bacteria further to ensure the recovery of only psychrotrophic bacteria, the bacteria are subjected to growth, on media such as PAF, in a cold environment. More particularly, the bacteria are grown at three different temperatures; 4°C, 10°C and 14°C. Those bacteria exhibiting growth, e.g. an observable lawn within certain time periods, are recovered and screened further. Thus, those bacteria exhibiting growth within 24 hours at 14°C, within 48 hours at 10°C and within 5 days e.g. 3-5 days, at 4°C are selected.

EPR candidates are subsequently screened for growth on a medium which contains seed-exudate as the sole source of carbon and nitrogen. Preferably the exudate is derived from the seed to which the EPR are to be later applied. For example, screening of EPR for application to soybean seed will entail growth on soybean seed exudate medium and, similarly, canola EPR are screened on canola seed exudate medium. Having previously selected only psychrotrophs for examination at this stage of the screening process, ambient temperatures may be employed e.g. 20°C. Those bacteria exhibiting growth are selected and further screened in an emergence assay.

In assaying for the capability to promote emergence, an experimental group of seeds planted in the presence of an EPR candidate is grown and compared with a control population. Those bacteria which promote a 50% greater seedling population than the control on each or three consecutive days are deemed EPR. As a further criterion, only those bacteria which repeatedly e.g. at least twice in three trials, exhibit emergence promotion are preferably selected.

Those bacteria which can be selected using the described screening program are EPR bacteria. Specific examples of strains which meet the criteria are listed below in Table 1. These strains, including clones and sub-clones thereof comprise a preferred aspect of the present invention.

## TABLE 1

### Identification of Emergence Promoting Bacteria

#### Soybeans

| Strain Designation | Identification | ATCC Accession # |
|---|---|---|
| 1-104 | Pseudomonas putida | |
| 1-226 | Pseudomonas fluorescens | |
| 1-206 | Pseudomonas fluorescens | |
| 2-16 | Serratia liquefaciens | |
| 2-18 | Serratia liquefaciens | |
| 2-20 | Serratia liquefaciens | |
| 2-22 | Pseudomonas putida biovar B | |
| 2-67 | Serratia liquefaciens | |
| 2-68 | Pseudomonas liquefaciens | |
| 2-114 | Serratia fonticola | 53450 |
| 17-114 | Pseudomonas putida | |
| 17-29 | Pseudomonas fluorescens | |
| 17-76 | Pseudomonas putida | |
| 17-34 | Pseudomonas fluorescens | |
| G25-25 | Pseudomonas fluorescens | |
| G25-26 | Pseudomonas putida | |
| G25-44 | Pseudomonas putida | 53451 |
| G20-20 | Pseudomonas fluorescens | |
| G25-34 | Pseudomonas putida | |
| G24-16 | Pseudomonas putida | |
| G24-14 | Pseudomonas putida | |
| G24-3 | Pseudomonas putida | |
| G20-18 | Pseudomonas fluorescens | 53449 |
| 1-102 | Serratia Proteamaculans ss. quinovora | 53287 |

| Strain Designation | Identification | ATCC Accession # |
|---|---|---|
| G1-1 | Beijerinckia indica* | 53447 |
| G1-3 | Pseudomonas fluorescens | |
| G1-4 | unknown | |
| 52-30 | Pseudomonas putida | |

\* tentative designation

Several of these bacteria were deposited with the American Type Culture Collection in Rockville, Maryland, U.S.A. The Accession numbers of those strains are given in the Table above. Strain 53287 was deposited on October 10, 1985. All other ATCC-deposited strains were deposited on January 27, 1986. All strains listed are maintained in a permanently viable condition in the culture collection of Allelix Inc., 6850 Goreway Drive, Mississauga, Ontario, Canada and will be made available to those authorized persons entitled to access to them in accordance with national or regional requirements.

Taxonomic characteristics of the strains are equivalent to the archetype of the species as described in Bergey's Manual of Determinative Bacteriology although each may be further characterized by growth on either canola or soybean seed exudate. The tentative designation given to strain G1-1 results from taxonomic studies which reveal that G1-1 most closely resembles Beijerinckia indica. Hence, that term is used throughout herein.

The discovery of plant growth promoting activity in a bacteria of the genus Serratia, or Beijerinckia, especially in the species Serratia liquefaciens, Serratia fonticola, Serratia proteamaculans and Beijerinckia indica, is novel and unexpected. Similarly, the discovery of EPR effective of such low soil temperatures is novel, and is of significant agricultural importance. At suboptimal soil temperatures, sedling emergence is reduced (Acharya, et. al., 1983; Szyrmer and Szczepanska, 1982), and seed exudation is increased. (Schroth, et. al., 1966; Keeling, 1974; Hayman, 1969). The EPR strains reported herein were selected for growth on seed exudates at low temperatures, and hence may serve to reduce the total carbohydrates in the spermosphere which are available for growth or seedling pathogens. Thus, EPR will indirectly stimulate yields under adverse growing conditions. The development of plant cultivars with increased emergence rates at low soil temperatures has previously been identified as a high priority for canola and soybean breeders in order to increase yields. In addition, EPR should prove useful as one component in an integrated management strategy to increase stands, since increased emergence rates at cold temperatures result in increased final stands and often in yield.

The invention is further described with reference to experimental methods and techniques used to isolate, identify and test the EPR of the present invention and with reference to the accompanying drawings in which

Figure 1 is a graphical representation of the results of an assay reported below, i.e. emergence promotion of canola with EPR in a greenhouse assay at 9°C;

Figure 2 is a similar graphical representation of a soybean assay reported below, i.e. emergency promotion of soybean with EPR in a greenhouse assay at 14°C;

Figure 3 is a graphical representation similar to Figure 2 but showing results of enhanced emergence and final stand of soybean with EPR in the greenhouse assay.

## Example 1 - Isolation of Bacterial Strains

Two different isolation procedures were used. For procedure one, canola and soybean seeds were surface-sterilized by rinsing for 5 minutes with 95% ethanol, followed by sterile water and 1.5% sodium hypochlorite for 5 minutes and were planted in various cold-climate-derived soil samples at 10 to 14°C. Roots from developing seedlings were removed, washed in sterile water to remove loosely adhering soil particle and ground in 5mL sterile 0.1 M $MgSO_4$. Serial ten-fold dilutions were plated onto Pseudomonas agar F (PAF) (Difco Labs, Detroit, MI, USA 48232) and plates were incubated 2 wks at 14°C. Colonies were purified on PAF at 20°C.

For isolation procedure two, roots of plants collected in the eastern Northwest Territories, Canada, were washed to remove soil particles and placed directly onto asparagine sort agar (ASA). ASA contained lg/L-asparagine, 2g Bacto Agar and 1000 mL distilled water. Bacteria which grew out from root segments on ASA were then purified on PAF plates at 20°C.

Strains isolated using procedures one and two were restreaked on PAF plates, and examined for rapid growth at 4, 10 and 14°C. Strains which developed an observable lawn in 24h at 14°C, 48h at 10°C and 3-5 days at 4°C were further tested for growth on exudate agar at 20°C.

Exudate agar was prepared by mixing 10% soybean or 20% canola seed exudates with 2% washed purified agar (Difco). Exudates were prepared as described by Scher et al, 1985.

Using isolation procedure one, 630 strain were obtained from soybean and 450 from canola. Of these strains, 277 soybean and 50 canola strains were found to grow on PAF in 3-5 days at 4°C and on exudate agars in 24hr at 20°C.

With isolation procedure two, 940 strains were obtained by direct isolation of chemotactic zones from roots on asparagine soft agar. Approximately 150 or these grew on PAF in 3-5 days at 4°C.

The second procedure for isolation of candidate EPR strains, in which root segments were placed directly onto asparagine soft agar, allowed the direct isolation of motile strains which were chemotactically attracted to one of the major amino acids in seed exudates, (Scher et al., 1985). This procedure yielded a higher percentage of strains which were ultimately deemed to be EPR strains based on repeatible emergence promotion: for canola, 4 of 60 (7%) strains from procedure two versus 0 of 50 from procedure one; for soybean, 14 or 277 (5%) strains from procedure one versus 9 of 84 (11%) from procedure two.

## Example 2 - Canola Emergence Assay

The following assay was developed to assess emergence of canola (cv 'Tobin'). Field soil was collected from the Allelix Field Research Centre near Caledon, Ontario and consisted of a clay loam with 2% organic matter, pH 7.0, total exchange capacity (M.E.) 14, and with the following nutrient levels in ppm: nitrate nitrogen 4, phosphorous 1, potassium 2, calcium 70, magnesium 16, sodium 0.5, boron 0.4, iron 550, manganese 130, copper 2, and zinc 7. Soil was thoroughly mixed in a 1:1 ratio with perlite and the resulting mix was used throughout the study.

Test bacteria were grown on PAF places at 10°C for 3 days, scraped off plates, and mixed in 0.1 M $MgSO_4$. Canola seeds were agitated in the bacterial suspensions for 2hr at 10°C prior to planting 20 seeds in each of 8 replicate 15 cm pots. Seeds were planted 2 cm deep and pots were watered immediately and placed at 9°C. Each experiment consisted of 6 to 8 bacterial treatments with one control. The control consisted of canola seeds soaked in 0.1 M $MgSO_4$ which had been poured over an uninoculated PAF plate. Pots were examined daily and the number of emerged seedlings was recorded.

A total of 50 bacterial strains isolated using isolation procedure one and 60 strains isolated using isolation procedure two were tested for emergence promotion relative to controls. Strains which demonstrated significant (P = 0.05) emergence promotion were retested 3 times to determine the consistency of emergence promotion.

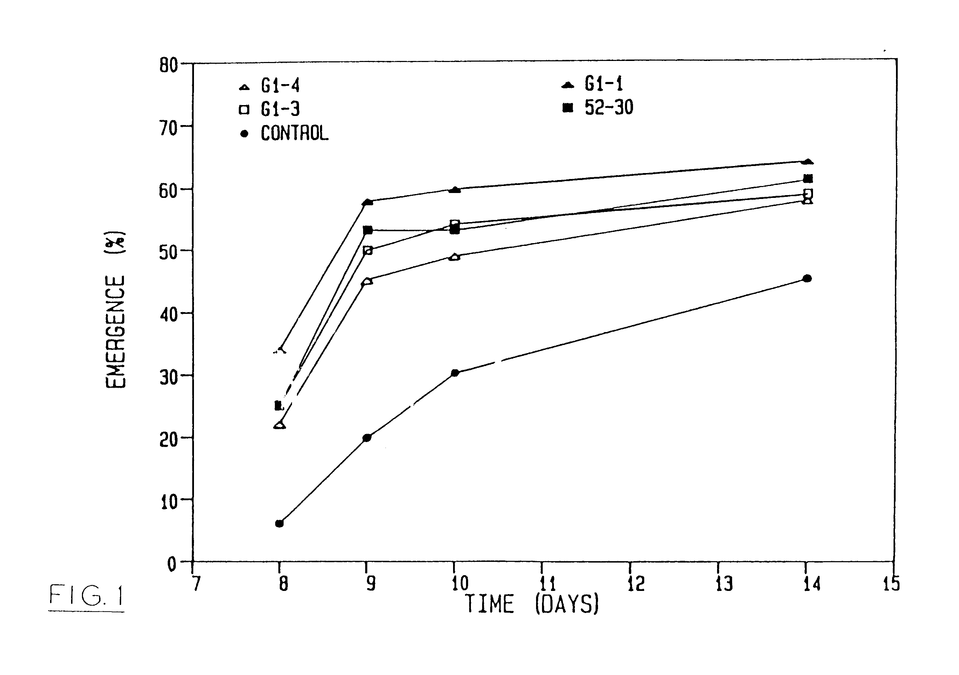

Of 50 strains obtained using isolation procedure one, 3 induced increases in emergence of 50% or greater than controls in first tests. None or these 3 increased emergence in 3 repeat experiments. Sixty strains obtained using isolation procedure two were tested in the canola assay and 10 increased emergence 40% or more in first trials. Four strains consistently increased emergence in 3 of 5 repeat trials. Fig. 1 graphically illustrates these results. It plots as vertical axis the number of emerged plants, against time as horizontal axis, using the 4 EPR strains and a control. The assay was conducted in a field soil:perlite mix at 9°C with seeds sown to a depth of 2 cm. The percentage emergence values shown are the mean of 8 replications, each sown with 20 seeds. Similar results were obtained with the same 4 strains in 3 of 5 repeat experiments. The percentage of emerged seedlings with bacterial treatments was 4 to 7 times greater than the percentage of emerged controls 8 days after seeding, 2 to 3 times greater at 9 days and 40 to 50% greater at 14 days.

## Example 3 - Soybean Emergence Assay

In the initial assay, candidate EPR strains were grown for 48hr on PAF plates at 14°C and scraped into 50 mL 0.1 M $MgSO_4$. One hundred fifty soybean seeds (cv. 'Maple Presto' or 'Maple Arrow') were added to each 50 mL suspension and were shaken at 100 RPM at 10°C for 3h. Typical experiments consisted of 6 bacterial treatments with one $MgSO_4$ control, each with 9 to 10 replications. Each replication consisted of 12 seeds planted in a 12-well plastic seeding tray (Plant Products Ltd., Bramalea, Ontario) with overall dimensions of 18 cm wide X 27 cm long X 6 cm deep and with dimensions or individual wells or 6 cm long X 5 cm wide X 6 cm deep. Seeds were planted 3 cm deep in "conditioned field soil".

"Conditioned field soil" was prepared by mixing soil from the Allelix Field Research Centre (described previously) in a 1:5 ratio with Promix C (Plant Products Ltd., Bramalea, Ontario). Soybean was seeded into flats containing the soil mixture and grown to the second true leaf stage when the plants were discarded. The same soil (termed "conditioned soil") was reblended and used in the soybean emergence assay.

After planting, each replicate seeding tray was watered and placed at 14°C. The number of emerged seedlings was recorded after 14 days, and data were analysed using a one-way analysis of variance to detect sigfnificant differences between treatment means. Strains which induced a significant increase in emergence in the first trial were retested twice using the same assay procedures.

A second assay was used subsequently for strains which demonstrated repeated EPR activity in the initial assay. Soybean seeds were shaken in bacterial suspensions or in 0.1 M $MgSO_4$ as described above and planted in a 1:1 mix of Allelix field soil:perlite. Five cm of the mix was placed in the bottom of 25 cm azalea pots (Kord Plastics Ltd., Toronto); 20 seeds of the same treatment were placed on the soil; soil perlite mix was added to give a planting depth of 5 cm. Pots were immediately watered and placed at 14°C. Each pot of 20 seeds constituted a single replication, and 8 replications were used per treatment. Typical experiments consisted of 5 to 7 bacterial treatments with a 0.1 M $MgSO_4$ control. Emergence was recorded daily and strains were deemed "EPR" when they induced a 50% increase in emergence of controls for 3 consecutive days in 2 or 3 repeating experiments.

Over a 9 month period, 277 strains isolated using procedure one and 84 strains isolated using procedure two were tested in the initial soybean emergence assay using "conditioned field soil". Sixty-two strains induced significant increases in emergence at 14 days compared to controls. Raw data from one typical experiment in which 2 EPR strains were selected, are shown in Table 2. All 62 strains were retested twice with the same assay and 30 strains repeated emergence promotion in both repeat experiments. Raw data from a typical repeat experiment is shown in Table 3.

## Table 2

### Soybean emergence assay

### Initial Selection Untested Strains*

#### Number emerged/12 at 14 days
#### Replication

| Treatment | 1 | 2 | 3 | 4 | 5 | 6 | 7 | 8 | 9 | 10 | x |
|---|---|---|---|---|---|---|---|---|---|---|---|
| 1 | 2 | 4 | 2 | 3 | 2 | 5 | 3 | 3 | 2 | 4 | 3.0** |
| 2 | 1 | 3 | 1 | 2 | 0 | 2 | 1 | 3 | 1 | 0 | 1.4 |
| 3 | 1 | 2 | 1 | 0 | 1 | 1 | 2 | 1 | 0 | 1 | 1.0 |
| 4 | 2 | 2 | 3 | 2 | 3 | 2 | 3 | 5 | 2 | 3 | 2.7** |
| 5 | 1 | 2 | 2 | 1 | 0 | 2 | 1 | 1 | 1 | 0 | 1.1 |
| 6 | 0 | 1 | 1 | 2 | 3 | 1 | 1 | 2 | 0 | 1 | 1.2 |
| Control | 2 | 2 | 1 | 1 | 1 | 0 | 3 | 1 | 2 | 1 | 1.4 |

LSD 0.01 = 1.1          F = 8.1

\* Assay was conducted at 14°C. The data shown are from one typical experiment. A total of 361 strains were tested over a 9 month period. See text for details.

Each treatment 1-6 is with seeds treated with a different bacterial strain. Each replication involves treatment of 12 seeds with the selected bacterial strain.

### Table 3

Soybean emergence assay - repeat testing of strains which induced a significant increase in emergence in the first test.*

**Number emerged/12 at 14 days**
**Replication**

| Treatment | 1 | 2 | 3 | 4 | 5 | 6 | 7 | 8 | 9 | 10 | x |
|-----------|---|---|---|---|---|---|---|---|---|----|---|
| 2-16 | 1 | 0 | 7 | 3 | 3 | 3 | 1 | 3 | 2 | 1 | 2.4* |
| 2-17 | 6 | 1 | 0 | 1 | 1 | 0 | 2 | 4 | 3 | 6 | 2.4* |
| 2-18 | 3 | 1 | 5 | 2 | 9 | 3 | 6 | 9 | 5 | 8 | 5.1** |
| 2-19 | 1 | 3 | 4 | 1 | 0 | 1 | 3 | 1 | 3 | 8 | 2.5* |
| 2-20 | 5 | 6 | 1 | 1 | 2 | 4 | 2 | 1 | 4 | 0 | 2.6* |
| 2-21 | 2 | 5 | 5 | 2 | 4 | 5 | 4 | 5 | 2 | 7 | 4.1** |
| Control | 0 | 1 | 1 | 1 | 2 | 0 | 0 | 0 | 0 | 0 | 0.5 |

LSD 0.05 = 1.8          F = 4.94

0.01 = 2.5

\* Assay procedures were the same used for data shown in Table 2. A total of 62 strains were retested. Data shown here are from one typical experiment. Data from all experiments were used for final selection of EPR strains.

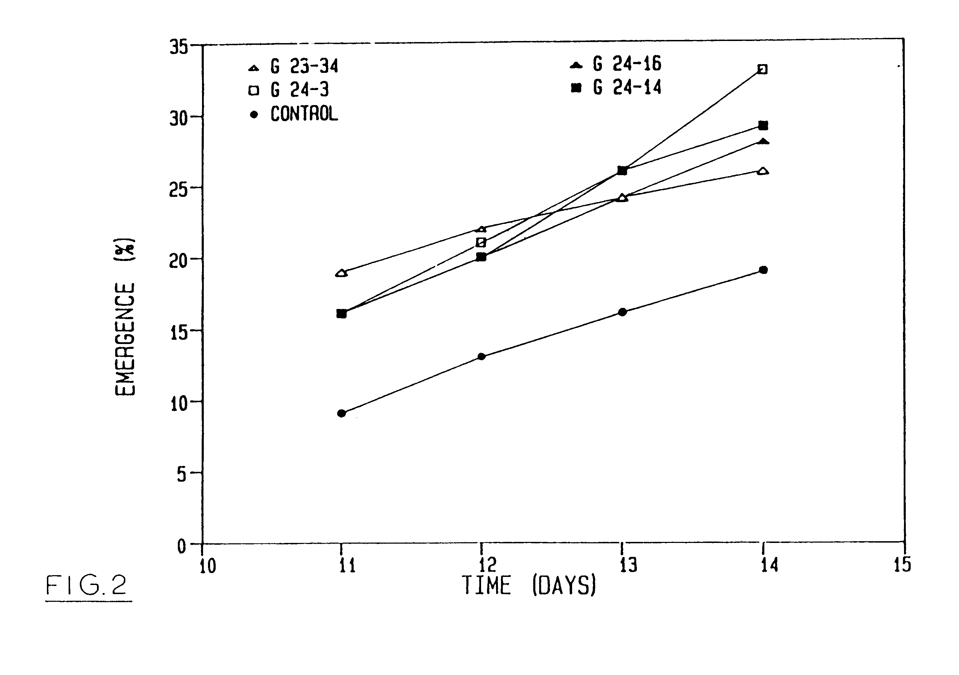

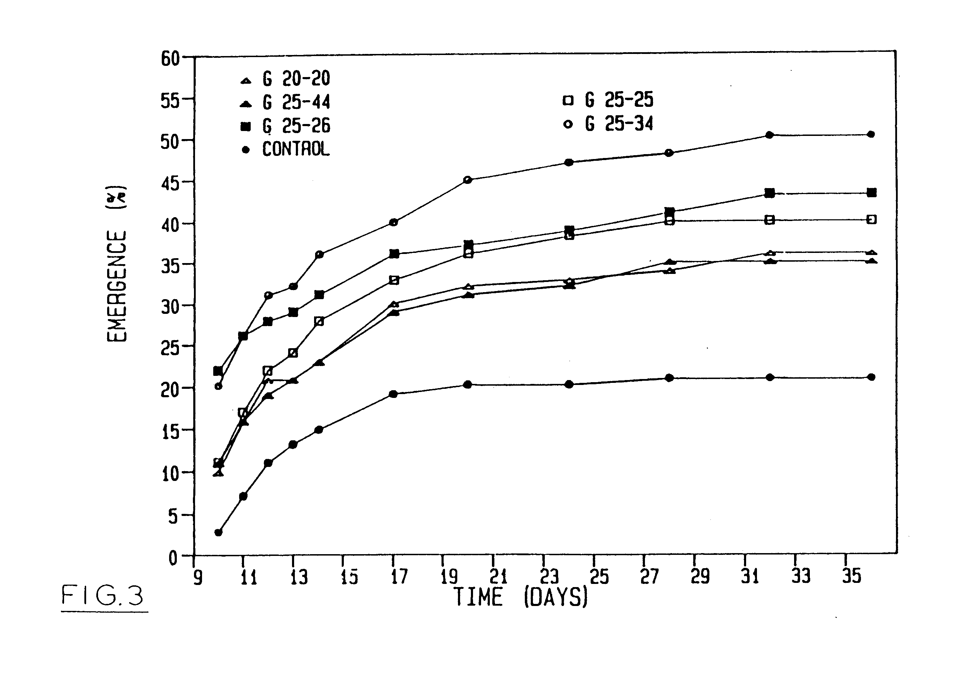

A second assay in field soil:perlite with 20 seeds per replication was used over a 12 month period to confirm the phenomenon of emergence promotion and to determine the effect of EPR on emergence on multiple days. Strains which induced a 50% increase in the percentage emergence of controls on each of 3 consecutive days were deemed to be EPR. Fig. 2 shows graphically the results of soybean emergence promotion, showing increases in emergence by 4 EPR strains. The assay was conducted in a field soil:perlite mix at 14°C. It is the second described assay. The percentage emergence values shown are the means of 8 replications, each sown with 20 seeds. Twenty-three of the 30 strains which were originally selected for emergence-promoting activity repeated emergence promotion in at least 2 of 3 repeat experiments. Some of these strains increased both the rate of emergence and the final percentage emergence under the experimental conditions. Fig. 3 illustrates these results, showing the effect of 5 soybean EPR on early emergence rate and final percentage emergence. The assay procedures are the same as used in connection with Fig. 2 results.

Example 4 - Identification and Storage of Bacteria

Purified bacterial strains were stored in glycerol at-80°C prior to being tested in the assays. Strains which induced emergence increases were rechecked for purity on PAFand 10 copies of each strain were

returned to -80°C storage. A new vial of bacteria was used for each emergence assay.

Identification was done only on strains which repeated emergence-promoting activity. All strains tested Gram-stain negative and were further tested for reaction profiles on API 20E test strips (Analytab Products, Ayerst Laboratories, Inc., Plainview, N.Y., U.S.A.). Additional tests included growth on MacConkey medium, type of metabolism in OF glucose medium, production of fluorescent pigment, gelatin hydrolysis, nitrate, reduction, starch hydrolysis, oxidase reaction, production of DNase, and lipase production (Tween 80 hydrolysis). Methods for all of the above biochemical tests were those recommended by the American Society for Microbiology. The identification of emergence promoting strains obtained from the described sources are listed in Table 1, given above.

The preferred method for detecting emergence promotion is the second soybean assay in which a field soil, perlite mixture was used and emergence was recorded daily. It was also found important to conduct each assay 3 times and select strains which increase emergence in 2 of 3 tests.

In the work described herein, a strain is designated an EPR if it induced about a 50% or greater increase in emergence relative to controls on 3 consecutive days. Alternative selection parameters are probably equally valid, as long as the emphasis is placed upon demonstrating the repeatibility of emergence promotion.

Example 5 - Field Trials

To establish the capacity of the isolated bacteria to promote seedling emergence (canola and soybean) and to test for capacity to increase seed yields in the field, under natural conditions, field trials were conducted. A total of 22 soybean EPR and 5 canola EPR strains were tested.

In a first series of trials soybean seeds cv. 'Maple Arrow' or 'Evans' were hand planted in a field near Caledon, Ontario, Canada after soaking in bacterial (EPR) suspensions ($10^9$ cfu/ml). Twenty seeds were planted per metre of a 4 metre plot, each plot being replicated 8 times using the Latin Square Design. Seeds were planted 3-6 cm deep and allowed to grow under natural conditions.

The results of 12 trials, using different EPR strains, are noted below in tables 4-7. In each case, a positive response (" + ") indicates that a statistically significant increase in emergence relative to controls was observed on three consecutive counting periods. "Vigour" is based on a visual rating of 1 to 10 and was taken 45 to 55 days after planting. Seed yield was adjusted to 10% moisture.

The details of the emergence response from one representative trial are shown in Table 5. The corresponding vigour and yield results for this representative trial are presented in Table 4.

## Table 4

Soybean EPR strains - cultivar 'Evans'
(suspension treated seeds)

| EPR Strain | Emergence Response[1] (see further Table 5 | Vigour Rating[2] | Yield (kg/ha)[3] | % increase compared to control |
|---|---|---|---|---|
| 1-206 | + | 7.8 | 1782 | 8 |
| 1-104 | + | 8.1 | 1739 | 5 |
| 1-226 | + | 8.9* | 1883 | 14 |
| 2-20 | + | 7.5 | 1696 | 3 |
| 2-16 | + | 7.4 | 1835 | 11 |
| 2-22 | + | 8.1 | 1922* | 17* |
| 2-18 | + | 8.0 | 1883 | 14 |
| control | | 7.1 | 1653 | - |

*, ** significant increase compared to control at P = 0.10, 0.05

Table 5

Effects of EPR strains on Emergence Incidence
of 'Evans' Soybean

Percentage Emergence
Days after Planting

| Strain | 14 | 15 | 16 | 17 | 20 | 23 | 24 | 27 | 30 |
|---|---|---|---|---|---|---|---|---|---|
| 1-226 | 0.8 | 1.9 | 4.4 | 15.5 | 36.7* | 54.2*** | 58.1*** | 65.9*** | 70.2*** |
| 2-22 | 0.7 | 1.3 | 4.1 | 15.9 | 39.0* | 55.0*** | 59.1*** | 64.5*** | 68.6*** |
| 1-104 | 1.3 | 2.8 | 5.0 | 13.6 | 38.0* | 53.6** | 59.2*** | 65.8*** | 68.0*** |
| 2-18 | 0.5 | 0.6 | 3.0 | 11.9 | 33.3* | 49.4** | 52.7*** | 61.7*** | 64.5*** |
| 1-206 | 0.5 | 1.1 | 3.0 | 15.0 | 35.5* | 48.4** | 54.4*** | 61.4*** | 64.4*** |
| 2-20 | 0.3 | 1.3 | 3.3 | 13.6 | 31.1* | 45.6* | 50.8*** | 57.7*** | 61.4*** |
| 2-16 | 0.2 | 0.9 | 3.1 | 13.8 | 29.5 | 43.3* | 46.9** | 55.3** | 59.1** |
| Control | 0 | 0 | 0.8 | 4.5 | 17.0 | 26.4 | 30.3 | 41.3 | 44.1 |

* ** *** indicates statistically significant increase compared to the control at P = 0.10, P = 0.05 and P = 0.01 respectively.

Table 6 shows the results obtained when treating soybean seeds of cultivar "Maple Arrow". Table 7 shows results of similar experiments on soybean cultivar "Evans".

## Table 6

### Soybean EPR strains - Cultivar 'Maple Arrow' (suspension treatment)

| EPR Strain | Emergence Response | Vigour Rating | Yield (Kg/ha) | % Yield Increase Compared to Control |
|---|---|---|---|---|
| 1-206 | + | 8.6** | 1491 | 9 |
| 1-104 | + | 8.8** | 1452 | 6 |
| 1-226 | + | 9.4** | 1590** | 17** |
| 2-20 | + | 8.9** | 1432 | 5 |
| 2-16 | + | 8.5** | 1488 | 9 |
| 2-22 | + | 8.5** | 1435 | 5 |
| 2-18 | + | 8.4** | 1515* | 11* |
| Control | + | 6.9 | 1366 | - |
| 17-114 | + | 7.6 | 1112 | 10 |
| 2-67 | + | 7.4 | 1188 | 18 |
| 2-68 | + | 8.5** | 1204* | 20* |
| 17-29 | + | 9.3** | 1300** | 29** |
| control | | 6.6 | 1006 | - |
| G20-18 | + | 8.9** | 1399** | 20** |
| 1-102 | + | 8.5** | 1412** | 21** |
| G24-3 | + | 7.3 | 1317 | 13 |
| G24-16 | + | 7.3 | 1218 | 5 |
| G25-34 | + | 8.1** | 1264 | 9 |

| EPR Strain | Emergence Response | Vigour Rating | Yield (Kg/ha) | % Yield Increase Compared to Control |
|---|---|---|---|---|
| G20-20 | + | 8.9** | 1346 | 16 |
| G24-14 | + | 9.0** | 1379* | 18* |
| control | | 6.5 | 1165 | - |
| 1-104 | + | 6.8 | 1175 | 3 |
| 1-226 | + | 7.4** | 1218 | 7 |
| 1-206 | + | 6.4 | 1277 | 12 |
| 2-16 | + | 7.0* | 1264 | 11 |
| 2-18 | + | 7.5** | 1379* | 21* |
| 2-22 | + | 7.0* | 1152 | 1 |
| control | | 5.6 | 1138 | - |
| 2-67 | + | 6.5** | 1000** | 26** |
| 2-68 | + | 5.8** | 993* | 25* |
| 17-29 | + | 6.6** | 954 | 20 |
| 17-76 | + | 5.5** | 953 | 20 |
| 17-34 | + | 7.0*< | 1181** | 49** |
| control | | 4.1 | 792 | - |
| G20-20 | + | 6.0** | 556* | 23* |
| G23-34 | + | 6.0** | 620** | 35** |
| G24-16 | + | 6.0** | 561 | 21 |
| G24-14 | + | 6.1** | 587** | 27** |
| G24-3 | + | 6.3** | 591** | 28** |
| G20-18 | + | 6.3** | 567* | 23* |
| 1-102 | + | 6.4** | 709** | 54** |
| control | | 4.3 | 462 | - |

*,** significant increase compared to control at P=0.10, 0.05

## Table 7

### Soybean EPR strains - Cultivar 'Evans'
### (suspension treatment)

| EPR<br>Strain | Emergence<br>Response | Vigour<br>Rating | Yield<br>(Kg/ha) | % Yield<br>Increase<br>Compared to<br>Control |
|---|---|---|---|---|
| G20-18 | + | 8.3** | 1822** | 21** |
| 1-102 | + | 8.6** | 1855** | 23** |
| G24-16 | + | 8.3** | 1675 | 11 |
| G25-34 | + | 8.3** | 1676 | 11 |
| G20-20 | + | 8.6** | 1696 | 13 |
| Control | | 6.0 | 1505 | - |
| 1-104 | + | 8.4** | 1501** | 19* |
| 1-226 | + | 8.6** | 1615** | 27** |
| 1-206 | + | 7.0 | 1330 | 5 |
| 2-16 | + | 8.6** | 1435 | 13 |
| 2-18 | + | 8.6** | 1409 | 11 |
| 2-20 | + | 8.5** | 1472* | 16* |
| 2-22 | + | 8.4** | 1336 | 5 |
| Control | | 6.1 | 1267 | - |
| 2-114 | + | 7.6** | 954 | 16 |
| 17-114 | + | 7.3** | 977 | 19 |
| 17-29 | + | 8.1** | 1105** | 34** |
| 17-76 | + | 8.3** | 1049* | 27* |
| 17-34 | + | 7.0** | 927 | 12 |
| Control | | 5.5 | 825 | - |
| G20-20 | + | 7.8* | 1181 | 11 |

| EPR Strain | Emergence Response | Vigour Rating | Yield (Kg/ha) | % Yield Increase Compared to Control |
|------------|--------------------|---------------|---------------|--------------------------------------|
| G23-34 | + | 8.8** | 1050 | -1 |
| G24-14 | + | 9.1** | 1280** | 20** |
| G24-3 | + | 8.1** | 1171 | 10 |
| G20-18 | + | 7.9* | 1056 | -1 |
| 1-102 | + | 8.3** | 1181 | 11 |
| Control | | 6.6 | 1066 | - |

*, ** significant increase compared to control at P = 0.10, 0.05

In the trials which generated the results appearing in Tables 8 and 9 below, soybean seeds were mechanically planted after treatment with a "lyo/oil" preparation. The preparation was made as follows: bacteria were grown for 48 hours in tryptic soy broth at 28°C, centrifuged, washed twice and lyophilized in 2% mannitol. The lyophilized powder was mixed with soybean oil at a rate of 0.2g powder/mL oil. The resulting lyo/oil mix was mixed with soybean seed at a rate of 3mL/kg seed. The resulting bacterial population on the seed was determined by replicate plating and is given in the Tables. In planting, the plots consisted of 3m long rows with 2 rows per plot, 20 seeds/m rows, and 8 replications of each treatment. Field design was an 11 x 8 randomized complete block.

Table 8

| Soybean EPR strains - cultivar 'Evans' (lyo/oil treatment) | | | | | |
|------------|----------------------------|----------------------|---------------|------------------------------------|------------------------------------|
| EPR Strain | Bacterial population on seed[1] | Emergence Response | Yield (kg/ha) | % increase compared to oil control | % increase compared to untreated control |
| 2-16 | 5.2 | + | 1679 | 2 | 16* |
| 2-18 | 6.0 | + | 1692 | 3 | 17** |
| 2-20 | 5.4 | + | 1683 | 3 | 16* |
| 2-67 | 4.6 | + | 1738 | 6 | 20** |
| 2-68 | 5.8 | + | 1617 | -1 | 12 |
| G25-44 | 4.5 | + | 1786 | 9 | 23** |
| G24-3 | 2.6 | + | 1657 | 1 | 14* |
| G20-18 | 2.3 | + | 1773 | 8 | 22** |
| 1-102 | 5.3 | + | 1767 | 8 | 22** |
| oil control | 0 | - | 1641 | - | 13 |
| untreated control | 0 | - | 1449 | -12 | - |

[1]$\log_{10}$ colony forming units per seed of inoculated strain
*, ** significant increase compared to the indicated control at P = 0.10, 0.05

17

## Table 9

Soybean EPR strains - Cultivar 'Maple Arrow'
(lyo/oil treatment)

| EPR Strain | Bacterial Population, on Seed[1] | Emergence Response | Yield (Kg/ha) | % Increase Compared to Oil Control | % Increase Compared to Untreated Control |
|---|---|---|---|---|---|
| 2-16 | 5.3 | + | 1221 | 17 | 28* |
| 2-18 | 5.3 | + | 1201 | 14 | 26* |
| 2-20 | 5.1 | + | 1237 | 18 | 30* |
| 2-67 | 4.1 | + | 1224 | 17 | 29* |
| 2-68 | 5.1 | + | 1270 | 21 | 33** |
| G20-18 | 2.3 | + | 1175 | 12 | 24 |
| 1-102 | 5.0 | - | 1121 | 6 | 17 |
| oil control 10 | | 0 | - | 1046 | - |
| untreated control | 0 | | 950 | -9 | - |

[1] Log[10] colony forming units per seed of innoculated strain.

*,** Significant increase compared to the indicated control
at P = 0.10, 0.05.

In the following Tables 10 - 13, the trials which generated the tabulated results were conducted on canola planted in order to generate and collect emergence data only. Plots were 1m long (8 replicates), randomized complete block design, and the seeds were suspension treated and hand planted in rows at a density of 40 seeds per metre row. Two cultivars were used i.e. "Tobin" (Brassica campestris) and "Triton" (Brassica napus).

18

## Table 10

Canola EPR strains – cultivar 'Tobin' suspension treatment

Analysis for Emergence
Percentage Emergence
DAYS AFTER PLANTING

| Strain | 6 | 6.5 | 7 | 7.5 | 8 | 8.5 | 9 | 9.5 | 10 |
|---|---|---|---|---|---|---|---|---|---|
| G1-3 | 8.8*** | 14.7*** | 18.1*** | 26.9*** | 34.1*** | 37.8*** | 41.9*** | 41.3*** | 43.3*** |
| 52-30 | 6.6* | 10.6* | 15.6*** | 25.0*** | 29.1*** | 38.1*** | 40.9*** | 41.9*** | 44.4*** |
| G1-1 | 9.1*** | 13.8*** | 17.8*** | 24.7*** | 28.4*** | 31.9*** | 35.9*** | 38.4*** | 41.9*** |
| G1-4 | 8.4*** | 12.2*** | 15.6*** | 19.7*** | 26.6*** | 32.2*** | 35.9*** | 36.9*** | 40.3*** |
| G2-9 | 7.5** | 10.6** | 13.4** | 18.8** | 21.9*** | 29.7*** | 33.8*** | 37.2*** | 39.4*** |
| Control | 2.8 | 4.1 | 5.9 | 9.4 | 13.4 | 18.1 | 21.6 | 22.8 | 24.7 |

* ** ***   indicates statistically significant increase compare to controls
on the indicated day at P = 0.10, 0.05 and 0.01

## Table 11

Canola EPR strains - cultivar 'Tobin' suspension treatment

Analysis for Emergence

Percentage Emergence

DAYS AFTER PLANTING

| Strain | 8 | 9 | 9.5 | 10 | 10.5 | 11 | 11.5 | 12 | 14 |
|--------|-----|------|------|-------|--------|--------|--------|---------|---------|
| G1-3 | 1.3 | 8.1 | 13.4 | 19.7* | 25.0** | 28.1** | 29.1** | 37.2*** | 59.7*** |
| 52-30 | 2.8 | 10.3 | 13.8 | 21.8* | 25.6** | 29.4** | 32.2** | 35.9*** | 56.0*** |
| G1-4 | 1.0 | 5.6 | 8.4 | 15.3 | 18.1 | 20.6 | 25.3 | 32.5** | 53.1*** |
| G1-1 | 1.9 | 5.9 | 9.1 | 14.4 | 17.5 | 20.6 | 24.4 | 30.6* | 54.1*** |
| G2-9 | 2.5 | 9.7 | 12.5 | 15.9 | 19.7 | 21.9 | 24.1 | 28.1 | 48.8* |
| Control | 0.6 | 2.8 | 5.3 | 9.7 | 12.8 | 15.0 | 17.8 | 22.8 | 38.1 |

\* \*\* \*\*\* indicates statistically significant increase compared to controls

on the indicated day at P = 0.10, 0.05 and 0.01

## Table 12

Canola EPR strains - cultivar 'Tobin' suspension treatment

Analysis for emergence

Percentage Emergence

DAYS AFTER PLANTING

| Strain | 7 | 8 | 8.5 | 9.5 | 10 | 10.5 | 11 |
|--------|------|------|------|------|------|------|------|
| G1-1 | 14.1*** | 28.8*** | 36.6*** | 50.7*** | 52.8*** | 56.3*** | 57.8*** |
| G1-3 | 10.3*** | 26.3*** | 31.6*** | 44.4*** | 47.8*** | 50.0*** | 51.9*** |
| G1-4 | 10.3*** | 17.8*** | 26.3*** | 43.1*** | 46.6*** | 48.8*** | 49.1** |
| G2-9 | 7.8** | 24.4*** | 33.1*** | 52.9*** | 52.2*** | 54.4*** | 56.3*** |
| 52-30 | 7.5** | 20.0*** | 27.8*** | 41.4*** | 49.4*** | 50.9*** | 53.4*** |
| Control | 0.9 | 5.9 | 11.3 | 18.1 | 25.9 | 30.6 | 35.6 |

\* \*\* \*\*\*  indicates statistically significant increase compared to controls on the indicated day at P = 0.10, 0.05, 0.01

EP 0 228 457 B1

## Table 13

Canola EPR strains - cultivar 'Triton' suspension treatment

Analysis for Emergence

Percentage Emergence

DAYS AFTER PLANTING

| STRAIN | 7.0 | 8.0 | 8.5 | 9.0 | 9.5 | 10.0 | 11.0 | 12.0 | 14.0 | 19.0 |
|--------|-----|-----|-----|-----|-----|------|------|------|------|------|
| G2-9 | 10.6*** | 24.7*** | 28.4*** | 38.4*** | 43.4*** | 49.4*** | 52.2*** | 55.9*** | 57.8*** | 59.1*** |
| G1-4 | 7.2** | 18.4*** | 26.3*** | 36.3*** | 39.7*** | 44.4*** | 45.6*** | 46.6*** | 50.3*** | 51.3*** |
| 52-30 | 7.8*** | 20.9*** | 24.7*** | 31.3** | 36.3*** | 39.7*** | 41.3** | 43.1** | 44.1*** | 45.6** |
| G1-1 | 4.7* | 12.2 | 16.6 | 24.7 | 30.9* | 35.3* | 39.1* | 42.8* | 44.7*** | 45.3** |
| G1-3 | 4.7* | 15.3** | 20.3** | 25.9* | 26.9 | 31.9 | 33.8 | 36.9 | 37.8 | 40.0 |
| Control | 0.6 | 5.9 | 9.7 | 15.9 | 19.1 | 23.4 | 25.9 | 27.5 | 30.0 | 32.5 |

* ** *** indicates statistically significant increase compared to controls on the indicated day at P = 0.10, 0.05, 0.01.

In the remaining Tables 14 and 15, the trials were conducted for yield analysis by planting canola seeds treated with bacterial suspensions in 4m long rows, 40 seeds/m, 8 replicates, using a randomized complete block design.

## Table 14

### Canola EPR Strains - Cultivar 'Tobin' - Suspension Treatment Analysis for Percentage Emergence[1]

#### DAYS AFTER PLANTING

| STRAIN | 11 | 12 | 12.5 | 13 | 14 | 17 |
|---|---|---|---|---|---|---|
| 52-30 | 33.8* | 37.3** | 38.6** | 39.5** | 39.9** | 40.4** |
| G1-1 | 29.6* | 34.3** | 36.3** | 37.5** | 38.1** | 38.8** |
| G2-9 | 30.5* | 32.3** | 34.8** | 36.7** | 37.5** | 38.3** |
| G1-4 | 25.8 | 28.3 | 28.8 | 31.5* | 33.2* | 34.0* |
| G1-3 | 20.5 | 23.0 | ∠4.1 | 24.6 | 25.0 | 26.1 |
| control | 18.4 | 20.2 | ?).8 | 21.8 | 22.7 | 23.4 |

[1] Yield data for this trial is shown in Table 15.

*,**,*** Indicates statistically significant increase compared to controls on the indicated day at P = 0.10, 0.05, 0.01.

Table 15

| Canola EPR Strains - Cultivar 'Tobin' - Suspension Treatment Analysis for Yield[1] | | |
|---|---|---|
| EPR Strain | Yield[1] (Kg/ha) | % Increase Compared to Control |
| 52-30 | 1868* | 25* |
| G1-1 | 1960** | 31** |
| G2-9 | 1637 | 9 |
| G1-4 | 1637 | 9 |
| G1-3 | 1492 | 0 |
| Control | 1498 | - |

[1] Emergence data for this trial are shown in Table 14.
*,** Statistically significant increase compared to control at P = 0.10, 0.05.

The Tables appearing above confirm that the ability to increase seed emergence via inoculation with EPR strains, which was observed in greenhouse assays, also occurs under natural conditions in field trials. In addition, under field conditions, this emergence benefit frequently translates to a statistically enhanced plant vigour and yield.

It will be noted that these EPR have been developed primarily in association with soybean and canola crops. However it will be appreciated that their utility is not necessarily restricted thereto, and the scope of the present invention extends to cover the growth promotion of other agriculturally significant crops using EPR according to the present invention.

**Claims**

23

**Claims for the following Contracting States : DE, FR, GB, IT**

1. A method for identifying bacteria capable of promoting emergence of seedlings from plant seed when applied to the growth environment of the seed, which comprises

   collecting bacteria which colonize plant roots;

   selecting those collected bacteria exhibiting growth under psychrotrophic conditions and growth on media containing seed exudate as the sole carbon source;

   growing seedlings from a population of seed grown in soil at temperatures tolerable by psychrotrophic bacteria wherein the growth environment of an experimental portion of the seed population contains an effective population of one of the selected bacteria and the growth environment of the remaining portion contains no selected bacteria; and

   selecting those bacteria added to the growth environment of the experimental seed population exhibiting an at least 50% greater number of emerged seedlings by comparison with the control population each day for at least three consecutive days during a seedling emergence period.

2. The method according to claim 1 wherein the bacteria indigenous to plant roots are collected from seedlings grown from soil indigenous to climates in which psychrotrophic conditions are encountered.

3. The method according to claim 2 wherein the bacteria exhibiting growth under psychrotrophic conditions are those bacteria exhibiting growth within the following time periods at the following temperatures: growth within 24 hours at 14°C; growth within 48 hours at 10°C; and growth within 5 days at 4°C.

4. The method according to any one of claims 1, 2 or 3 wherein the exudate in the seed exudate medium on which the bacteria are cultured is exudate characteristic of the species of seed for which the bacteria are intended to promote emergence.

5. The method according to any preceding claim wherein the seed exudate-containing medium contains canola seed exudate.

6. The method according to any preceding claim wherein the seed exudate-containing medium contains soybean seed exudate.

7. A biologically pure culture of a novel, psychrotrophic rhizobacterium capable of promoting emergence of seedlings from plant seed and exhibiting growth on seed exudate-containing medium wherein the sole carbon and nitrogen source in said medium is said seed exudate.

8. A biologically pure culture of an emergence promoting rhizobacterium identified by the method defined in any one of claims 1-6.

9. A biologically pure culture of an emergence promoting rhizobacterium as defined in claim 7 or claim 8 which is designated herein as G1-1 having ATCC accession number 53447, clones and sub-clones thereof.

10. A biologically pure culture of an emergence promoting rhizobacterium as defined in claim 7 or claim 8 which is designated herein as Pseudomonas fluorescens G20-18 having ATCC accession number 53449, clones and sub-clones thereof.

11. A biologically pure culture of an emergence promoting rhizobacterium as defined in claim 7 or claim 8 which is designated herein as Serratia fonticola 2-114 having ATCC accession number 53450, clones and sub-clones thereof.

12. A biologically pure culture of an emergence promoting rhizobacterium as defined in claim 7 or claim 8 which is designated herein as Pseudomonas putida G-25-44 having ATCC accession number 53451, clones and sub-clones thereof.

13. A plant emergence promoting composition comprising a psychrotrophic rhizobacterium capable of promoting emergence of seedlings from plant seed and exhibiting growth on medium containing seed exudate as the sole carbon source and an agronomically acceptable carrier.

14. The composition according to claim 13 wherein the rhizobacterium is present in the carrier at a concentration sufficient to deliver from $10^2$ and $10^9$ rhizobacteria to the spermosphere of an individual seed.

15. The composition according to claim 13 wherein the rhizobacterium is present in the carrier at a concentration effective to promote growth when applied to the seeds before planting.

16. The composition according to claim 15 wherein the rhizobacterium is present in the carrier at a concentration sufficient to deliver from $10^3$ to $10^6$ rhizobacteria to the spermosphere of an individual seed.

17. Seed having coated thereon an emergence promoting rhizobacterium as defined in any one of claims 7 to 12 in a population per seed of from $10^2$ to $10^9$ cells.

18. Seed according to claim 17 wherein said seed is canola seed and the bacteria coated thereon are selected from among the group of EPR bacteria species Beijerinckia indicia, Pseudomonas fluorescens, and Pseudomonas putida.

19. Seed according to claim 17 wherein said seed is soybean seed and the bacteria are selected from

| Strain Designation | Identification | ATCC Accession # |
|---|---|---|
| 1-104 | Pseudomonas putida | |
| 1-226 | Pseudomonas fluorescens | |
| 1-206 | Pseudomonas fluorescens | |
| 2-16 | Serratia liquefaciens | |
| 2-18 | Serratia liquefaciens | |
| 2-20 | Serratia liquefaciens | |
| 2-22 | Pseudomonas putida biovar B | |
| 2-67 | Serratia liquefaciens | |
| 2-68 | Pseudomonas liquefaciens | |
| 2-114 | Serratia fonticola | 53450 |
| 17-114 | Pseudomonas putida | |
| 17-29 | Pseudomonas fluorescens | |
| 17-76 | Pseudomonas putida | |
| 17-34 | Pseudomonas fluorescens | |
| G25-25 | Pseudomonas fluorescens | |
| G25-26 | Pseudomonas putida | |
| G25-44 | Pseudomonas putida | 53451 |
| G20-20 | Pseudomonas fluorescens | |
| G25-34 | Pseudomonas putida | |
| G24-16 | Pseudomonas putida | |
| G24-14 | Pseudomonas putida | |
| G24-3 | Pseudomonas putida | |
| G20-18 | Pseudomonas fluorescens | 53449 |
| 1-102 | Serratia proteamaculans ss. quinovora | 53287 |

20. A process for promoting the growth of agricultural crops at soil temperatures conducive to the growth of psychrotrophic bacteria which comprises applying to the growth environment of the crop an effective amount of at least one viable psychotrophic EPR bacterium effective to promote the growth of said crop.

**21.** A process according to claim 20, wherein the bacterium is applied to the growth environment of the crop by application of a composition containing the EPR bacterium to the crop seeds prior to planting, by direct application of a composition containing the EPR bacterium to the soil of the growth environment either prior to or after planting the crop seeds therein, or by treating growing crop plants with a composition containing the EPR bacterium.

**22.** A process according to claim 21, wherein the crop is soybeans.

**23.** A process according to claim 22, wherein the bacterium EPR strain is selected from Pseudomonas putida, Pseudomonas putida biovar B, Serratia liquefaciens, Pseudomonas fluorescens and Serratia fonticola (ATCC-53450) and Serratia proteamaculans ss. quinovora (ATCC-53287).

**24.** A process according to claim 21, wherein the crop is canola.

**25.** A process according to claim 24, wherein the bacteria EPR strains is selected from Pseudomonas putida, Pseudomonas fluorescens and Beijerinckia indicia.

**Claims for the following Contracting States : SE, BE**

**1.** A method for identifying bacteria capable of promoting emergence of seedlings from plant seed when applied to the growth environment of the seed, which comprises

collecting bacteria which colonize plant roots;

selecting those collected bacteria exhibiting growth under psychrotrophic conditions and growth on media containing seed exudate as the sole carbon source;

growing seedlings from a population of seed grown in soil at temperatures tolerable by psychrotrophic bacteria wherein the growth environment of an experimental portion of the seed population contains an effective population of one of the selected bacteria and the growth environment of the remaining portion contains no selected bacteria; and

selecting those bacteria added to the growth environment of the experimental seed population exhibiting an at least 50% greater number of emerged seedlings by comparison with the control population each day for at least three consecutive days during a seedling emergence period.

**2.** The method according to claim 1 wherein the bacteria indigenous to plant roots are collected from seedlings grown from soil indigenous to climates in which psychrotrophic conditions are encountered.

**3.** The method according to claim 2 wherein the bacteria exhibiting growth under psychrotrophic conditions are those bacteria exhibiting growth within the following time periods at the following temperatures: growth within 24 hours at 14°C; growth within 48 hours at 10°C; and growth within 5 days at 4°C.

**4.** The method according to any one of claims 1, 2 or 3 wherein the exudate in the seed exudate medium on which the bacteria are cultured is exudate characteristic of the species of seed for which the bacteria are intended to promote emergence.

**5.** The method according to any preceding claim wherein the seed exudate-containing medium contains canola seed exudate.

**6.** The method according to any preceding claim wherein the seed exudate-containing medium contains soybean seed exudate.

**7.** A biologically pure culture of a novel, psychrotrophic rhizobacterium capable of promoting emergence of seedlings from plant seed and exhibiting growth on seed exudate-containing medium wherein the sole carbon and nitrogen source in said medium is said seed exudate.

**8.** A biologically pure culture of an emergence promoting rhizobacterium identified by the method defined in any one of claims 1-6.

**9.** A biologically pure culture of an emergence promoting rhizobacterium as defined in claim 7 or claim 8 which is designated herein as G1-1 having ATCC accession number 53447, clones and sub-clones thereof.

**10.** A biologically pure culture of an emergence promoting rhizobacterium as defined in claim 7 or claim 8 which is designated herein as Pseudomonas fluorescens G20-18 having ATCC accession number 53449, clones and sub-clones thereof.

**11.** A biologically pure culture of an emergence promoting rhizobacterium as defined in claim 7 or claim 8 which is designated herein as Serratia fonticola 2-114 having ATCC accession number 53450, clones and sub-clones thereof.

**12.** A biologically pure culture of an emergence promoting rhizobacterium as defined in claim 7 or claim 8 which is designated herein as Pseudomonas putida G-25-44 having ATCC accession number 53451, clones and sub-clones thereof.

**13.** A biologically pure culture of an emergence promoting rhizobacterium as defined in claim 7 or claim 8 which is designated herein as Serratia proteamaculans ss. quinovora 1-102 having ATCC accession number 53287, clones and sub-clones thereof.

**14.** A plant emergence promoting composition comprising a psychrotrophic rhizobacterium capable of promoting emergence of seedlings from plant seed and exhibiting growth on medium containing seed exudate as the sole carbon source and an agronomically acceptable carrier.

**15.** The composition according to claim 14 wherein the rhizobacterium is present in the carrier at a concentration sufficient to deliver from $10^2$ and $10^9$ rhizobacteria to the spermosphere of an individual seed.

**16.** The composition according to claim 14 wherein the rhizobacterium is present in the carrier at a concentration effective to promote growth when applied to the seeds before planting.

**17.** The composition according to claim 16 wherein the rhizobacterium is present in the carrier at a concentration sufficient to deliver from $10^3$ to $10^6$ rhizobacteria to the spermosphere of an individual seed.

**18.** Seed having coated thereon an emergence promoting rhizobacterium as defined in any one of claims 7 to 13 in a population per seed of from $10^2$ to $10^9$ cells.

**19.** Seed according to claim 18 wherein said seed is canola seed and the bacteria coated thereon are selected from among the group of EPR bacteria species Beijerinckia indicia, Pseudomonas fluorescens, and Pseudomonas putida.

**20.** Seed according to claim 18 wherein said seed is soybean seed and the bacteria are selected from

27

EP 0 228 457 B1

| Strain Designation | Identification | ATCC Accession # |
|---|---|---|
| 1-104 | Pseudomonas putida | |
| 1-226 | Pseudomonas fluorescens | |
| 1-206 | Pseudomonas fluorescens | |
| 2-16 | Serratia liquefaciens | |
| 2-18 | Serratia liquefaciens | |
| 2-20 | Serratia liquefaciens | |
| 2-22 | Pseudomonas putida biovar B | |
| 2-67 | Serratia liquefaciens | |
| 2-68 | Pseudomonas liquefaciens | |
| 2-114 | Serratia fonticola | 53450 |
| 17-114 | Pseudomonas putida | |
| 17-29 | Pseudomonas fluorescens | |
| 17-76 | Pseudomonas putida | |
| 17-34 | Pseudomonas fluorescens | |
| G25-25 | Pseudomonas fluorescens | |
| G25-26 | Pseudomonas putida | |
| G25-44 | Pseudomonas putida | 53451 |
| G20-20 | Pseudomonas fluorescens | |
| G25-34 | Pseudomonas putida | |
| G24-16 | Pseudomonas putida | |
| G24-14 | Pseudomonas putida | |
| G24-3 | Pseudomonas putida | |
| G20-18 | Pseudomonas fluorescens | 53449 |
| 1-102 | Serratia proteamaculans ss. quinovora | 53287 |

**21.** A process for promoting the growth of agricultural crops at soil temperatures conducive to the growth of psychrotrophic bacteria which comprises applying to the growth environment of the crop an effective amount of at least one viable psychotrophic EPR bacterium effective to promote the growth of said crop.

**22.** A process according to claim 21, wherein the bacterium is applied to the growth environment of the crop by application of a composition containing the EPR bacterium to the crop seeds prior to planting, by direct application of a composition containing the EPR bacterium to the soil of the growth environment either prior to or after planting the crop seeds therein, or by treating growing crop plants with a composition containing the EPR bacterium.

**23.** A process according to claim 22, wherein the crop is soybeans.

**24.** A process according to claim 23, wherein the bacterium EPR strain is selected from Pseudomonas putida, Pseudomonas putida biovar B, Serratia liquefaciens, Pseudomonas fluorescens and Serratia fonticola (ATCC-53450) and Serratia proteamaculans ss. quinovora (ATCC-53287).

**25.** A process according to claim 22, wherein the crop is canola.

**26.** A process according to claim 25, wherein the bacteria EPR strains is selected from Pseudomonas putida, Pseudomonas fluorescens and Beijerinckia indicia.

**Patentansprüche**
**Patentansprüche für folgende Vertragsstaaten : DE, FR, GB, IT**

**1.** Verfahren zum Identifizieren von Bakterien, die dazu in der Lage sind, das Keimen von Saatgut von Pflanzensaat dann zu fördern, wenn auf die Wuchsumgebung des Saatgutes aufgebracht, umfassend:
Sammeln von Bakterien, die auf Pflanzenwurzeln Kolonien bilden;
Auswählen solcher gesammelter Bakterien, die unter psychrotrophischen Bedingungen wachsen sowie in Medien, welche Saat-Exsudat als einzige Kohlenstoffquelle enthalten;

28

Wachsenlassen des Saatgutes aus einer Population von Saatgut, das im Boden bei Temperaturen gewachsen ist, welche von psychrotrophischen Bakterien toleriert werden, wobei die Wuchsumgebung eines experimentellen Teiles der Saatpopulation eine wirksame Population eines der ausgewählten Bakterien enthält und die Wuchsumgebung des verbleibenden Teiles keine ausgewählten Bakterien enthält; und

Auswählen jener Bakterien, die der Wuchsumgebung der experimentellen Saatgutpopulation hinzugegeben wurden, die eine um wenigstens 50 % größere Anzahl von gekeimtem Saatgut entwickeln, durch täglichen Vergleich mit der Kontrollpopulation über wenigstens drei aufeinander folgende Tage während der Saatgutkeimperiode.

2. Verfahren nach Anspruch 1, wobei Bakterien, die bei Pflanzenwurzeln heimisch sind, für Saatgut gesammelt werden, welches in Böden gewachsen ist, die einem Klima angehören, in welchem psychrotrophische Bedingungen herrschen.

3. Verfahren nach Anspruch 2, wobei die Bakterien, welche unter psychrotrophischen Bedingungen Wachstum entwickeln, jene Bakterien sind, welche Wachstum innerhalb der folgenden Zeitspannen und bei den folgenden Temperaturen entwickeln: Wachstum innerhalb 24 Stunden bei 14°C; Wachstum innerhalb 48 Stunden bei 10°C; und Wachstum innerhalb von 5 Tagen bei 4°C.

4. Verfahren nach einem der Ansprüche 1, 2 oder 3, wobei das Exsudat im Saat-Exsudat-Mittel, auf welchem die Bakterien gezüchtet werden, ein Exsudat ist, das charakteristisch für die Spezies von Saatgut ist, für welche die Bakterien zum Fördern des Keimens bestimmt sind.

5. Verfahren nach einem der vorausgegangenen Ansprüche, wobei das saatgut-exsudat-enthaltende Medium Canola-Saatgut-Exsudat enthält.

6. Verfahren nach einem der vorausgegangenen Ansprüche, wobei das saatgut-exsudat-enthaltende Medium Sojabohnen-Saatgut-Exsudat enthält.

7. Biologisch reine Kultur eines neuen, psychrotrophischen Rhizobakterium, in der Lage, das Keimen von Saatgut aus Pflanzen-Saatgut zu fördern und Wachstum im saatgut-exsudat-enthaltenden Medium zu fördern, wobei die einzige Kohlenstoff- und Stickstoffquelle in dem genannten Medium das genannte Saatgut-Exsudat ist.

8. Biologisch reine Kultur eines keimungsfördernden Rhizobakterium, identifiziert durch das Verfahren definiert in einem der Ansprüche 1-6.

9. Biologisch reine Kultur eines keimungsfördernden Rhizobakterium, definiert in Anspruch 7 oder Anspruch 8, im folgenden bezeichnet als G1-1 mit der ATCC-Zugriffsnummer 53447, Clone und Unterclone hiervon.

10. Biologisch reine Kultur eines keimungsfördernden Rhizobakterium, definiert in Anspruch 7 oder Anspruch 8, im folgenden bezeichnet als Pseudomonas fluorescens G20-18, mit der ATCC-Zugriffsnummer 53449, Clone und Unterclone hiervon.

11. Biologisch reine Kultur eines keimungsfördernden Rhizobakterium, definiert in Anspruch 7 oder Anspruch 8, im folgenden bezeichnet als Serratia fonticola 2-114, mit der ATCC-Zugriffsnummer 53450, Clone und Unterclone hiervon.

12. Biologisch reine Kultur eines keimungsfördernden Rhizobakterium, definiert in Anspruch 7 oder Anspruch 8, im folgenden bezeichnet als Pseudomonas putida G25-44, mit der ATCC-Zugriffsnummer 53451, Clone und Unterclone hiervon.

13. Verbindung zum Fördern des Keimens von Pflanzen, mit einem psychrotrophischen Rhizobakterium, das dazu in der Lage ist, das Keimen von Saatgut aus Pflanzensaat zu fördern und Wachstum in einem Medium zu entwickeln, das Saatgut-Exsudat als einzige Kohlenstoffquelle sowie einen landwirtschaftlich zulässigen Träger enthält.

**14.** Verbindung gemäß Anspruch 13, wobei das Rhizobakterium in dem Träger bei einer Konzentration vorliegt, die ausreicht, um zwischen $10^2$ und $10^9$ Rhizobakterien an die Samenumgebung eines einzelnen Samenkornes abzugeben.

**15.** Verbindung gemäß Anspruch 13, wobei das Rhizobakterium im Träger bei einer Konzentration vorliegt, die wirksam ist, das Wachstum dann zu fördern, wenn auf das Saatgut vor dem Pflanzen aufgebracht.

**16.** Verbindung gemäß Anspruch 15, wobei das Rhizobakterium im Träger in einer Konzentration vorliegt, die ausreicht, um zwischen $10^3$ bis $10^6$ Rhizobakterien an die Samenumgebung des einzelnen Saatkornes abzugeben.

**17.** Saatgut, das mit einem die Keimung fördernden Rhizobakterium beschichtet ist, so wie in einem der Ansprüche 7 - 12, in einer Population pro Samenkorn von $10^2$ bis $10^9$ Zellen.

**18.** Saatgut gemäß Anspruch 17, wobei das Saatgut Canola-Saat ist, und die dieses beschichtenden Bakterien ausgewählt sind aus der Gruppe von EPR-Bakterien Beijerinckia indicia, Pseudomonas fluorescens und Pseudomonas putida.

**19.** Saatgut gemäß Anspruch 17, wobei das Saatgut Sojabohnen-Saatgut ist, und die Bakterien ausgewählt sind aus

| Stammbezeichnung | Identifikation | ATCC-Zugriffsnr. |
|---|---|---|
| 1-104 | Pseudomonas putida | |
| 1-226 | Pseudomonas fluorescens | |
| 1-206 | Pseudomonas fluorescens | |
| 2-16 | Serratia liquefaciens | |
| 2-18 | Serratia liquefaciens | |
| 2-20 | Serratia liquefaciens | |
| 2-22 | Pseudomonas putida biovar B | |
| 2-67 | Serratia liquefaciens | |
| 2-68 | Pseudomonas liquefaciens | |
| 2-114 | Serratia fonticola | 53450 |
| 17-114 | Pseudomonas putida | |
| 17-29 | Pseudomonas fluorescens | |
| 17-76 | Pseudomonas putida | |
| 17-34 | Pseudomonas fluorescens | |
| G25-25 | Pseudomonas fluorescens | |
| G25-26 | Pseudomonas putida | |
| G25-44 | Pseudomonas putida | 53451 |
| G20-20 | Pseudomonas fluorescens | |
| G25-34 | Pseudomonas putida | |
| G24-16 | Pseudomonas putida | |
| G24-14 | Pseudomonas putida | |
| G24-3 | Pseudomonas putida | |
| G20-18 | Pseudomonas fluorescens | 53449 |
| 1-102 | Serratia proteamaculans ss. quinovora | 53287 |

**20.** Verfahren zum Fördern des Wachstums von Getreide bei Bodentemperaturen, die dem Wachstum psychrotrophischer Bakterien förderlich sind, wobei auf die Wuchsumgebung des Getreides eine wirksame Menge wenigstens einer lebensfähigen phsychrotrophischen EPR-Bakterie aufgebracht wird, die wirksam ist, um das Wachstum des genannten Getreides zu fördern.

**21.** Verfahren gemäß Anspruch 20, wobei die auf die Wuchsumgebung aufgebrachten Bakterien des Getreides durch Aufbringen einer Verbindung, welche EPR-Bakterien enthält, auf das Getreidesaatgut vor dem Pflanzen aufgebracht werden, und zwar durch direktes Aufbringen einer Verbindung, welche EPR-Bakterien enthält, auf den Boden der Wuchsumgebung, entweder vor oder nach dem Pflanzen des Getreidesaatgutes oder durch Behandeln der wachsenden Getreidepflanzen mit einer Verbindung, welche die EPR-Bakterien enthält.

**22.** Verfahren nach Anspruch 21, wobei das Getreide Sojabohnen sind.

**23.** Verfahren nach Anspruch 22, wobei der Bakterienstamm ausgewählt ist aus Pseudomonas putida, Pseudomonas putida biovar B, Serratia liquefaciens, Pseudomonas fluorescens und Serratia fonticcia (ATCC-Zugriffsnummer 53450) und Serratia proteamaculans ss. quinovora (ATCC-Zugriffsnummer 53287).

**24.** Verfahren gemäß Anspruch 21, wobei das Getreide Canola ist.

**25.** Verfahren gemäß Anspruch 24, wobei die Bakterienstämme ausgewählt sind aus Pseudomonas putida, Pseudomonas fluorescens und Beijerinckia indicia.

**Patentansprüche für folgende Vertragsstaaten : SE, BE**

**1.** Verfahren zum Identifizieren von Bakterien, die dazu in der Lage sind, das Keimen von Saatgut von Pflanzensaat dann zu fördern, wenn auf die Wuchsumgebung des Saatgutes aufgebracht, umfassend:
Sammeln von Bakterien, die auf Pflanzenwurzeln Kolonien bilden;
Auswählen solcher gesammelter Bakterien, die unter psychrotrophischen Bedingungen wachsen sowie in Medien, welche Saat-Exsudat als einzige Kohlenstoffquelle enthalten;
Wachsenlassen des Saatgutes aus einer Population von Saatgut, das im Boden bei Temperaturen gewachsen ist, welche von psychrotrophischen Bakterien toleriert werden, wobei die Wuchsumgebung eines experimentellen Teiles der Saatpopulation eine wirksame Population eines der ausgewählten Bakterien enthält und die Wuchsumgebung des verbleibenden Teiles keine ausgewählten Bakterien enthält; und
Auswählen jener Bakterien, die der Wuchsumgebung der experimentellen Saatgutpopulation hinzugegeben wurden, die eine um wenigstens 50 % größere Anzahl von gekeimtem Saatgut entwickeln, durch täglichen Vergleich mit der Kontrollpopulation über wenigstens drei aufeinander folgende Tage während der Saatgutkeimperiode.

**2.** Verfahren nach Anspruch 1, wobei Bakterien, die bei Pflanzenwurzeln heimisch sind, für Saatgut gesammelt werden, welches in Böden gewachsen ist, die einem Klima angehören, in welchem psychrotrophische Bedingungen herrschen.

**3.** Verfahren nach Anspruch 2, wobei die Bakterien, welche unter psychrotrophischen Bedingungen Wachstum entwickeln, jene Bakterien sind, welche Wachstum innerhalb der folgenden Zeitspannen und bei den folgenden Temperaturen entwickeln: Wachstum innerhalb 24 Stunden bei 14°C; Wachstum innerhalb 48 Stunden bei 10°C; und Wachstum innerhalb von 5 Tagen bei 4°C.

**4.** Verfahren nach einem der Ansprüche 1, 2 oder 3, wobei das Exsudat im Saat-Exsudat-Mittel, auf welchem die Bakterien gezüchtet werden, ein Exsudat ist, das charakteristisch für die Spezies von Saatgut ist, für welche die Bakterien zum Fördern des Keimens bestimmt sind.

**5.** Verfahren nach einem der vorausgegangenen Ansprüche, wobei das saatgut-exsudat-enthaltende Medium Canola-Saatgut-Exsudat enthält.

**6.** Verfahren nach einem der vorausgegangenen Ansprüche, wobei das saatgut-exsudat-enthaltende Medium Sojabohnen-Saatgut-Exsudat enthält.

**7.** Biologisch reine Kultur eines neuen, psychrotrophischen Rhizobakterium, in der Lage, das Keimen von Saatgut aus Pflanzen-Saatgut zu fördern und Wachstum im saatgut-exsudat-enthaltenden Medium zu fördern, wobei die einzige Kohlenstoff- und Stickstoffquelle in dem genannten Medium das genannte Saatgut-Exsudat ist.

**8.** Biologisch reine Kultur eines keimungsfördernden Rhizobakterium, identifiziert durch das Verfahren definiert in einem der Ansprüche 1-6.

**9.** Biologisch reine Kultur eines keimungsfördernden Rhizobakterium, definiert in Anspruch 7 oder Anspruch 8, im folgenden bezeichnet als G1-1 mit der ATCC-Zugriffsnummer 53447, Clone und Unterclone hiervon.

**10.** Biologisch reine Kultur eines keimungsfördernden Rhizobakterium, definiert in Anspruch 7 oder Anspruch 8, im folgenden bezeichnet als Pseudomonas fluorescens G20-18, mit der ATCC-Zugriffsnummer 53449, Clone und Unterclone hiervon.

**11.** Biologisch reine Kultur eines keimungsfördernden Rhizobakterium, definiert in Anspruch 7 oder Anspruch 8, im folgenden bezeichnet als Serratia fonticola 2-114, mit der ATCC-Zugriffsnummer 53450, Clone und Unterclone hiervon.

**12.** Biologisch reine Kultur eines keimungsfördernden Rhizobakterium, definiert in Anspruch 7 oder Anspruch 8, im folgenden bezeichnet als Pseudomonas putida G25-44, mit der ATCC-Zugriffsnummer 53451, Clone und Unterclone hiervon.

**13.** Biologisch reine Kultur eines keimungsfördernden Rhizobakteriums gemäß Anspruch 7 oder 8, das hierbei als Serratia proteamaculans ss. quinovora 1-102 bezeichnet werden, mit der ATCC-Zugriffsnummer 53287, Clone und Unterclone hiervon.

**14.** Pflanzenkeimungs-fördernde Verbindung mit einem psychrotrophischen Rhizobakterium, das dazu in der Lage ist, die Keimung von Saatgut aus Pflanzensaat zu fördern und Wachstum auf einem Mediumenthaltenden Saat-Exsudat als einziger Kohlenstoffquelle und einem landwirtschaftlich akzeptablen Träger zu entwickeln.

**15.** Verbindung gemäß Anspruch 14, wobei das Rhizobakterium im Träger bei einer Konzentration vorliegt, die ausreicht, um zwischen $10^2$ und $10^9$ Rhizobakterien an die Samenumgebung des einzelnen Saatkornes abzugeben.

**16.** Verbindung gemäß Anspruch 15, wobei das Rhizobakterium im Träger in einer Konzentration vorliegt, die ausreicht, um zwischen $10^3$ und $10^6$ Rhizobakterien an die Samenumgebung des einzelnen Saatkornes abzugeben.

**17.** Verbindung gemäß Anspruch 16, wobei das Rhizobakterium im Träger in einer Konzentration vorliegt, die ausreicht, um zwischen $10^3$ und $10^6$ Rhizobakterien an die Samenumgebung des einzelnen Saatkornes abzugeben.

**18.** Saatgut, das mit einem die Keimung fördernden Rhizobakterium beschichtet ist, gemäß einem der Ansprüche 7-13 in einer Population pro Samenkorn von $10^2$ bis $10^9$ Zellen.

**19.** Saatgut gemäß Anspruch 18, wobei das Saatgut Canola-Saat ist und die diese beschichtenden Bakterien ausgewählt sind aus der Gruppe von EPR-Bakterien Beijerinckia indicia, Pseudomonas fluorescens, und Pseudomonas putida.

**20.** Saatgut gemäß Anspruch 18, wobei das Saatgut Sojabohnen-Saatgut ist, und die Bakterien ausgewählt sind aus

EP 0 228 457 B1

| Stammbezeichnung | Identifikation | ATCC-Zugriffsnr. |
|---|---|---|
| 1-104 | Pseudomonas putida | |
| 1-226 | Pseudomonas fluorescens | |
| 1-206 | Pseudomonas fluorescens | |
| 2-16 | Serratia liquefaciens | |
| 2-18 | Serratia liquefaciens | |
| 2-20 | Serratia liquefaciens | |
| 2-22 | Pseudomonas putida biovar B | |
| 2-67 | Serratia liquefaciens | |
| 2-68 | Pseudomonas liquefaciens | |
| 2-114 | Serratia fonticola | 53450 |
| 17-114 | Pseudomonas putida | |
| 17-29 | Pseudomonas fluorescens | |
| 17-76 | Pseudomonas putida | |
| 17-34 | Pseudomonas fluorescens | |
| G25-25 | Pseudomonas fluorescens | |
| G25-26 | Pseudomonas putida | |
| G25-44 | Pseudomonas putida | 53451 |
| G20-20 | Pseudomonas fluorescens | |
| G25-34 | Pseudomonas putida | |
| G24-16 | Pseudomonas putida | |
| G24-14 | Pseudomonas putida | |
| G24-3 | Pseudomonas putida | |
| G20-18 | Pseudomonas fluorescens | 53449 |
| 1-102 | Serratia proteamaculans ss. quinovora | 53287 |

**21.** Verfahren zum Fördern des Wachstums von Getreide bei Bodentemperaturen, die dem Wachstum psychrotrophischer Bakterien förderlich sind, wobei auf die Wuchsumgebung des Getreides eine wirksame Menge wenigstens einer lebensfähigen phsychrotrophischen EPR-Bakterie aufgebracht wird, die wirksam ist, um das Wachstum des genannten Getreides zu fördern.

**22.** Verfahren gemäß Anspruch 21, wobei die auf die Wuchsumgebung aufgebrachten Bakterien des Getreides durch Aufbringen einer Verbindung, welche EPR-Bakterien enthält, auf das Getreidesaatgut vor dem Pflanzen aufgebracht werden, und zwar durch direktes Aufbringen einer Verbindung, welche EPR-Bakterien enthält, auf den Boden der Wuchsumgebung, entweder vor oder nach dem Pflanzen des Getreidesaatgutes oder durch Behandeln der wachsenden Getreidepflanzen mit einer Verbindung, welche die EPR-Bakterien enthält.

**23.** Verfahren nach Anspruch 22, wobei das Getreide Sojabohnen sind.

**24.** Verfahren nach Anspruch 23, wobei der EPR-Bakterienstamm ausgewählt ist aus Pseudomonas putida, Pseudomonas putida biovar B, Serratia liquefaciens, Pseudomonas fluorescens und Serratia fonticola und Serratia proteamaculans ss. quinovora.

**25.** Verfahren nach Anspruch 22, wobei das Getreide Canola ist.

**26.** Verfahren nach Anspruch 25, wobei die EPR-Bakterienstämme ausgewählt sind aus Pseudomonas putida, Pseudomonas fluorescens und Beijerinckia indicia.

**Revendications**
**Revendications pour les Etats contractants suivants : DE, FR, GB, IT**

**1.** Procédé d'identification de bactéries capables de favoriser la levée de plantes de semis à partir de graines végétales lorsqu'elles sont appliquées à l'environnement où croissent les graines, qui comprend :
  le recueil de bactéries colonisant les racines de plantes ;

33

EP 0 228 457 B1

la sélection des bactéries recueillies faisant preuve de croissance dans des conditions psychrotrophes et de croissance sur des milieux contenant de l'exsudat des graines comme seule source de carbone ;

la culture des plantes de semis à partir d'une population de graines ayant poussé dans un sol à des températures tolérables par des bactéries psychrotrophes, l'environnement où croît une partie expérimentale de la population de graines contenant une population efficace d'une des bactéries sélectionnées et l'environnement où croît la partie résiduelle ne contenant pas de bactéries sélectionnées ; et

la sélection des bactéries ajoutées à l'environnement où croît la population expérimentale de graines faisant preuve d'un nombre de plantes de semis levées supérieur d'au moins 50 % par comparaison à la population témoin chaque jour pendant au moins 3 j consécutifs durant une période de levée des plantes de semis.

2. Procédé selon la revendication 1, dans lequel les bactéries indigènes aux racines végétales sont recueillies à partir de plantes de semis ayant poussé dans un sol indigène à des climats dans lesquels on rencontre des conditions psychrotrophes.

3. Procédé selon la revendication 2, dans lequel les bactéries faisant preuve de croissance dans des conditions psychrotrophes sont les bactéries faisant preuve de croissance en l'espace des durées suivantes aux températures suivantes : croissance en 24 h à 14ºC ; croissance en 48 h à 10ºC ; et croissance en 5 j à 4ºC.

4. Procédé selon l'une quelconque des revendications 1, 2 ou 3, dans lequel l'exsudat dans le milieu d'exsudat de graines sur lequel les bactéries sont cultivées est un exsudat caractéristique de l'espèce des graines dont les bactéries ont pour but de favoriser la levée.

5. Procédé selon l'une quelconque des revendications précédentes, dans lequel le milieu contenant de l'exsudat de graines contient de l'exsudat de graines de canola.

6. Procédé selon l'une quelconque des revendications précédentes, dans lequel le milieu contenant de l'exsudat de graines contient de l'exsudat de graines de soja.

7. Culture biologiquement pure d'une nouvelle rhizobactérie psychrotrophe capable de favoriser la levée de plantes de semis à partir de graines végétales et de faire preuve de croissance sur un milieu contenant de l'exsudat de graines, la seule source de carbone et d'azote dans ledit milieu étant ledit exsudat de graines.

8. Culture biologiquement pure d'une rhizobactérie favorisant la levée, identifiée par le procédé défini dans l'une quelconque des revendications 1 à 6.

9. Culture biologiquement pure d'une rhizobactérie favorisant la levée, telle que définie dans la revendication 7 ou la revendication 8, qui est désignée ici comme étant G1-1 ayant le numéro de dépôt ATCC 53447, ses clones et ses sous-clones.

10. Culture biologiquement pure d'une rhizobactérie favorisant la levée telle que définie dans la revendication 7 ou la revendication 8, qui est désignée ici comme étant Pseudomonas fluorescens G20-18 ayant le numéro de dépôt ATCC 53449, ses clones et ses sous-clones.

11. Culture biologiquement pure d'une rhizobactérie favorisant la levée telle que définie dans la revendication 7 ou la revendication 8, qui est désignée ici comme étant Serratia fonticola 2-114 ayant le numéro de dépôt ATCC 53450, ses clones et ses sous-clones.

12. Culture biologiquement pure d'une rhizobactérie favorisant la levée telle que définie dans la revendication 7 ou la revendication 8, qui est désignée ici comme étant Pseudomonas putida G-25-44 ayant le numéro de dépôt ATCC 53451, ses clones et ses sous-clones.

13. Composition favorisant la levée de plantes, comprenant une rhizobactérie psychrotrophe capable de favoriser la levée de plantes de semis à partir de graines végétales et de faire preuve de croissance

34

sur un milieu contenant de l'exsudat de graines comme seule source de carbone et un véhicule agronomiquement acceptable.

14. Composition selon la revendication 13, dans laquelle la rhizobactérie est présente dans le véhicule à une concentration suffisante pour fournir de $10^2$ à $10^9$ rhizobactéries à la spermosphère d'une graine individuelle.

15. Composition selon la revendication 13, dans laquelle la rhizobactérie est présente dans le véhicule à une concentration efficace pour favoriser la croissance lorsqu'elle est appliquée aux graines avant qu'elles soient plantées.

16. Composition selon la revendication 15, dans laquelle la rhizobactérie est présente dans le véhicule à une concentration suffisante pour fournir de $10^3$ à $10^6$ rhizobactéries à la spermosphère d'une graine individuelle.

17. Graines auxquelles est appliquée une rhizobactérie favorisant la levée telle que définie dans l'une quelconque des revendications 7 à 12 en une population par graine de $10^2$ à $10^9$ cellules.

18. Graines selon la revendication 17, dans lesquelles lesdites graines sont des graines de canola et les bactéries qui leur sont appliquées sont sélectionnées parmi le groupe des espèces bactériennes favorisant la levée Beijerinckia indicia, Pseudomonas fluorescens et Pseudomonas putida.

19. Graines selon la revendication 17, dans lesquelles lesdites graines sont des graines de soja et les bactéries sont sélectionnées parmi :

| Désignation de la souche | Identification | n° de dépôt ATCC |
|---|---|---|
| 1-104 | Pseudomonas putida | |
| 1-226 | Pseudomonas fluorescens | |
| 1-206 | Pseudomonas fluorescens | |
| 2-16 | Serratia liquefaciens | |
| 2-18 | Serratia liquefaciens | |
| 2-20 | Serratia liquefaciens | |
| 2-22 | Pseudomonas putida biovar B | |
| 2-67 | Serratia liquefaciens | |
| 2-68 | Pseudomonas liquefaciens | |
| 2-114 | Serratia fonticola | 53450 |
| 17-114 | Pseudomonas putida | |
| 17-29 | Pseudomonas fluorescens | |
| 17-76 | Pseudomonas putida | |
| 17-34 | Pseudomonas fluorescens | |
| G25-25 | Pseudomonas fluorescens | |
| G25-26 | Pseudomonas putida | |
| G25-44 | Pseudomonas putida | 53451 |
| G20-20 | Pseudomonas fluorescens | |
| G25-34 | Pseudomonas putida | |
| G24-16 | Pseudomonas putida | |
| G24-14 | Pseudomonas putida | |
| G24-3 | Pseudomonas putida | |
| G20-18 | Pseudomonas fluorescens | 53449 |
| 1-102 | Serratia proteamaculans ss. quinovora | 53287 |

20. Procédé destiné à favoriser la croissance de cultures agricoles à des températures du sol induisant la croissance de bactéries psychrotrophes, comprenant l'application à l'environnement où croît la culture d'une quantité efficace d'au moins une bactérie psychrotrophe viable favorisant la levée, efficace pour favoriser la croissance de ladite culture.

**21.** Procédé selon la revendication 20, dans lequel la bactérie est appliquée à l'environnement où croît la culture par application d'une composition contenant la bactérie favorisant la levée aux graines de la culture avant que celles-ci soient plantées, par application directe d'une composition contenant la bactérie favorisant la levée au sol de l'environnement où croît la culture soit avant soit après que les graines de la culture y aient été plantées, ou par traitement des plantes de la culture en cours de développement par une composition contenant la bactérie favorisant la levée.

**22.** Procédé selon la revendication 21, dans lequel la culture est du soja.

**23.** Procédé selon la revendication 22, dans lequel la souche bactérienne favorisant la levée est sélectionnée parmi Pseudomonas putida, Pseudomonas putida biovar B, Serratia liquefaciens, Pseudomonas fluorescens et Serratia fonticola (ATCC-53450) et Serratia proteamaculans ss. quinovora (ATCC-53287).

**24.** Procédé selon la revendication 21, dans lequel la culture est de la canola.

**25.** Procédé selon la revendication 24, dans lequel la souche bactérienne favorisant la levée est sélectionnée parmi Pseudomonas putida, Pseudomonas fluorescens et Beijerinckia indicia.

**Revendications pour les Etats contractants suivants : SE, BE**

**1.** Procédé d'identification de bactéries capables de favoriser la levée de plantes de semis à partir de graines végétales lorsqu'elles sont appliquées à l'environnement où croissent les graines, qui comprend :

le recueil de bactéries colonisant les racines de plantes ;

la sélection des bactéries recueillies faisant preuve de croissance dans des conditions psychrotrophes et de croissance sur des milieux contenant de l'exsudat de graines comme seule source de carbone ;

la culture des plantes de semis à partir d'une population de graines ayant poussé dans un sol à des températures tolérables par des bactéries psychrotrophes, l'environnement où croît une partie expérimentale de la population de graines contenant une population efficace d'une des bactéries sélectionnées et l'environnement où croît la partie résiduelle ne contenant pas de bactéries sélectionnées ; et

la sélection des bactéries ajoutées à l'environnement où croît la population expérimentale de graines faisant preuve d'un nombre de plantes de semis levées supérieur d'au moins 50 % par comparaison à la population témoin chaque jour pendant au moins 3 j consécutifs durant une période de levée des plantes de semis.

**2.** Procédé selon la revendication 1, dans lequel les bactéries indigènes aux racines végétales sont recueillies à partir de plantes de semis ayant poussé dans un sol indigène à des climats dans lesquels on rencontre des conditions psychrotrophes.

**3.** Procédé selon la revendication 2, dans lequel les bactéries faisant preuve de croissance dans des conditions psychrotrophes sont les bactéries faisant preuve de croissance en l'espace des durées suivantes aux températures suivantes : croissance en 24 h à 14°C ; croissance en 48 h à 10°C ; et croissance en 5 j à 4°C.

**4.** Procédé selon l'une quelconque des revendications 1, 2 ou 3, dans lequel l'exsudat dans le milieu d'exsudat de graines sur lequel les bactéries sont cultivées est un exsudat caractéristique de l'espèce des graines dont les bactéries ont pour but de favoriser la levée.

**5.** Procédé selon l'une quelconque des revendications précédentes, dans lequel le milieu contenant de l'exsudat de graines contient de l'exsudat de graines de canola.

**6.** Procédé selon l'une quelconque des revendications précédentes, dans lequel le milieu contenant de l'exsudat de graines contient de l'exsudat de graines de soja.

**7.** Culture biologiquement pure d'une nouvelle rhizobactérie psychrotrophe capable de favoriser la levée de plantes de semis à partir de graines végétales et de faire preuve de croissance sur un milieu contenant de l'exsudat de graines, la seule source de carbone et d'azote dans ledit milieu étant ledit exsudat de graines.

8. Culture biologiquement pure d'une rhizobactérie favorisant la levée, identifiée par le procédé défini dans l'une quelconque des revendications 1 à 6.

9. Culture biologiquement pure d'une rhizobactérie favorisant la levée, telle que définie dans la revendication 7 ou la revendication 8, qui est désignée ici comme étant G1-1 ayant le numéro de dépôt ATCC 53447, ses clones et ses sous-clones.

10. Culture biologiquement pure d'une rhizobactérie favorisant la levée telle que définie dans la revendication 7 ou la revendication 8, qui est désignée ici comme étant Pseudomonas fluorescens G20-18 ayant le numéro de dépôt ATCC 53449, ses clones et ses sous-clones.

11. Culture biologiquement pure d'une rhizobactérie favorisant la levée telle que définie dans la revendication 7 ou la revendication 8, qui est désignée ici comme étant Serratia fonticola 2-114 ayant le numéro de dépôt ATCC 53450, ses clones et ses sous-clones.

12. Culture biologiquement pure d'une rhizobactérie favorisant la levée telle que définie dans la revendication 7 ou la revendication 8, qui est désignée ici comme étant Pseudomonas putida G-25-44 ayant le numéro de dépôt ATCC 53451, ses clones et ses sous-clones.

13. Culture biologiquement pure d'une rhizobactérie favorisant la levée telle que définie dans la revendication 7 ou la revendication 8, qui est désignée ici comme étant Serratia proteamaculans ss. quinovora 1-102 ayant le numéro de dépôt ATCC 53287, ses clones et ses sous-clones.

14. Composition favorisant la levée de plantes, comprenant une rhizobactérie psychrotrophe capable de favoriser la levée de plantes de semis à partir de graines végétales et de faire preuve de croissance sur un milieu contenant de l'exsudat de graines comme seule source de carbone et un véhicule agronomiquement acceptable.

15. Composition selon la revendication 14, dans laquelle la rhizobactérie est présente dans le véhicule à une concentration suffisante pour fournir de $10^2$ à $10^9$ rhizobactéries à la spermosphère d'une graine individuelle.

16. Composition selon la revendication 14, dans laquelle la rhizobactérie est présente dans le véhicule à une concentration efficace pour favoriser la croissance lorsqu'elle est appliquée aux graines avant qu'elles soient plantées.

17. Composition selon la revendication 16, dans laquelle la rhizobactérie est présente dans le véhicule à une concentration suffisante pour fournir de $10^3$ à $10^6$ rhizobactéries à la spermosphère d'une graine individuelle.

18. Graines auxquelles est appliquée une rhizobactérie favorisant la levée telle que définie dans l'une quelconque des revendications 7 à 13 en une population par graine de $10^2$ à $10^9$ cellules.

19. Graines selon la revendication 18, dans lesquelles lesdites graines sont des graines de canola et les bactéries qui leur sont appliquées sont sélectionnées parmi le groupe des espèces bactériennes favorisant la levée Beijerinckia indicia, Pseudomonas fluorescens et Pseudomonas putida.

20. Graines selon la revendication 18, dans lesquelles lesdites graines sont des graines de soja et les bactéries sont sélectionnées parmi :

| Désignation de la souche | Identification | n° de dépôt ATCC |
|---|---|---|
| 1-104 | Pseudomonas putida | |
| 1-226 | Pseudomonas fluorescens | |
| 1-206 | Pseudomonas fluorescens | |
| 2-16 | Serratia liquefaciens | |
| 2-18 | Serratia liquefaciens | |
| 2-20 | Serratia liquefaciens | |
| 2-22 | Pseudomonas putida biovar B | |
| 2-67 | Serratia liquefaciens | |
| 2-68 | Pseudomonas liquefaciens | |
| 2-114 | Serratia fonticola | 53450 |
| 17-114 | Pseudomonas putida | |
| 17-29 | Pseudomonas fluorescens | |
| 17-29 | Pseudomonas fluorescens | |
| 17-76 | Pseudomonas putida | |
| 17-34 | Pseudomonas fluorescens | |
| G25-25 | Pseudomonas fluorescens | |
| G25-26 | Pseudomonas putida | |
| G25-44 | Pseudomonas putida | 53451 |
| G20-20 | Pseudomonas fluorescens | |
| G25-34 | Pseudomonas putida | |
| G24-16 | Pseudomonas putida | |
| G24-14 | Pseudomonas putida | |
| G24-3 | Pseudomonas putida | |
| G20-18 | Pseudomonas fluorescens | 53449 |
| 1-102 | Serratia proteamaculans ss. quinovora | 53287 |

21. Procédé destiné à favoriser la croissance de cultures agricoles à des températures du sol induisant la croissance de bactéries psychrotrophes, comprenant l'application à l'environnement où croît la culture d'une quantité efficace d'au moins une bactérie psychrotrophe viable favorisant la levée, efficace pour favoriser la croissance de ladite culture.

22. Procédé selon la revendication 21, dans lequel la bactérie est appliquée à l'environnement où croît la culture par application d'une composition contenant la bactérie favorisant la levée aux graines de la culture avant que celles-ci soient plantées, par application directe d'une composition contenant la bactérie favorisant la levée au sol de l'environnement où croît la culture soit avant soit après que les graines de la culture y aient été plantées, ou par traitement des plantes de la culture en cours de développement par une composition contenant la bactérie favorisant la levée.

23. Procédé selon la revendication 22, dans lequel la culture est du soja.

24. Procédé selon la revendication 23, dans lequel la souche bactérienne favorisant la levée est sélectionnée parmi Pseudomonas putida, Pseudomonas putida biovar B, Serratia liquefaciens, Pseudomonas fluorescens et Serratia fonticola et Serratia proteamaculans ss. quinovora.

25. Procédé selon la revendication 22, dans lequel la culture est de la canola.

26. Procédé selon la revendication 25, dans lequel la souche bactérienne favorisant la levée est sélectionnée parmi Pseudomonas putida, Pseudomonas fluorescens et Beijerinckia indicia.

FIG.1

EP 0 228 457 B1

FIG.2

EP 0 228 457 B1

FIG.3

EP 0 228 457 B1